# EUROPEAN PATENT APPLICATION

(11) **EP 4 653 009 A1**
(43) Date of publication of application: **26.11.2025**
(21) Application number: 24744393.0
(22) Date of filing: 19.01.2024
(51) Int. Cl.: A61K 38/17, C07K 16/28, C07K 19/00, A61K 39/395, A61P 37/00

(54) **METHOD FOR TREATING SYSTEMIC LUPUS ERYTHEMATOSUS**

(30) Priority: 19.01.2023 CN 202310086927
(71) Applicant: Shanghai Huaota Biopharmaceutical Co., Ltd., Shanghai 201203 (CN); Huabo Biopharm (Shanghai) Co., Ltd., Shanghai 201203 (CN)
(72) Inventor: ZHAN, Yifan, Shanghai 201203 (CN); FENG, Yujie, Shanghai 201203 (CN); MA, Xiaojuan, Shanghai 201203 (CN); ZHU, Xiangyang, Shanghai 201203 (CN)
(74) Representative: karo IP
(86) International application number: PCT/CN2024/073225
(87) International publication number: WO 2024/153223

(57) **Abstract**

The present application relates to the use of a drug combination in the preparation of a drug. The drug is used for preventing and/or treating systemic lupus erythematosus, and the drug combination comprises an antigen binding protein targeting IL-17A and an antigen binding protein targeting IL-36R. The present application further relates to the use of a bispecific antibody targeting IL-17A and targeting IL-36R in the preparation of a drug.

## Description

### TECHNICAL FIELD

The present application relates to the field of biomedicine, and specifically to a method for preventing and/or treating systemic lupus erythematosus by targeting IL-17A and IL-36R.

### BACKGROUND

Systemic lupus erythematosus (SLE) is a rheumatic immune disease that can cause irreversible damage to organs. Lupus nephritis is the main complication of SLE, manifested as proteinuria, hematuria, tubular urine, and progressive decline in renal function, and eventually even requires renal transplantation or long-term dialysis treatment, which seriously affects the quality of life of patients and is called "immortal cancer". Currently, there are limited treatment options, and more therapeutic drugs are urgently needed to meet clinical needs.

IL-17A is the main pro-inflammatory factor of the IL17 family. IL-17A is secreted by Th17, CD8+ (Tc17), γδT, NKT and ILC3s cells. IL36 is a member of the IL1 superfamily and consists of three agonists IL36α, IL36β, and IL36γ and a natural receptor antagonist IL-36Ra. Once activated, this pathway promotes the production of a plurality of inflammatory factors. Literature reports that IL17 is highly expressed in patients with active SLE, and animal models and clinical trial data show that blocking IL17 has the potential to treat SLE and lupus nephritis (LN). Currently, Novartis and Jiangsu Hengrui Pharmaceuticals Co., Ltd. are conducting Phase III clinical trials to investigate the efficacy of IL17 monoclonal antibodies in the treatment of LN. The levels of IL36α and IL36γ in the serum of SLE patients are significantly increased and positively correlated with the SLE disease activity index (SLEDAI).

However, at present, there is still an urgent need to develop more effective treatment options to reduce the mortality rate of patients, slow down the rate of increase in urine protein levels, and reduce renal pathological damage.

### SUMMARY

The present application provides a method for preventing and/or treating systemic lupus erythematosus by targeting IL-17A and IL-36R. In the present application, by jointly administering an antigen binding protein targeting IL-17A and an antigen binding protein targeting IL-36R, or by administering a bispecific antigen binding protein targeting IL-17A and IL-36R, the synergistic effect of the two targets can be exerted, thereby more effectively treating systemic lupus erythematosus compared with monotherapy targeting a single target alone. By targeting IL-17A and IL-36R, one or more of the following therapeutic effects can be achieved on systemic lupus erythematosus or diseases and/or conditions caused by systemic lupus erythematosus: (1) the increase in urine protein level of the subject can be inhibited; (2) the renal pathological damage in the subject can be significantly improved; and (3) the renal fibrosis in the subject can be inhibited.

In one aspect, the present application provides the use of a drug combination in the preparation of a drug for preventing and/or treating systemic lupus erythematosus, wherein the drug combination comprises an antigen binding protein targeting IL-17A and an antigen binding protein targeting IL-36R.

In certain embodiments, the IL-17A is human IL-17A.

In certain embodiments, the antigen binding protein targeting IL-17A is capable of blocking the related effects of IL-17A and IL-17R.

In certain embodiments, the antigen binding protein targeting IL-17A comprises at least one CDR from an antibody heavy chain variable region VH, and the VH comprises an amino acid sequence as set forth in SEQ ID NO: 16.

In certain embodiments, the antigen binding protein targeting IL-17A comprises HCDR3, and the HCDR3 comprises an amino acid sequence as set forth in SEQ ID NO: 13.

In certain embodiments, the antigen binding protein targeting IL-17A comprises HCDR2, and the HCDR2 comprises an amino acid sequence as set forth in SEQ ID NO: 14.

In certain embodiments, the antigen binding protein targeting IL-17A comprises HCDR1, and the HCDR1 comprises an amino acid sequence as set forth in SEQ ID NO: 15.

In certain embodiments, the antigen binding protein targeting IL-17A comprises VH, and the VH comprises an amino acid sequence as set forth in SEQ ID NO: 16.

In certain embodiments, the antigen binding protein targeting IL-17A comprises an antibody heavy chain constant region.

In certain embodiments, the antibody heavy chain constant region is derived from a heavy chain constant region of IgG.

In certain embodiments, the antibody heavy chain constant region is derived from a heavy chain constant region of IgG1 or IgG4.

In certain embodiments, the antibody heavy chain constant region of the antigen binding protein targeting IL-17A comprises an amino acid sequence as set forth in SEQ ID NO: 21.

In certain embodiments, the antigen binding protein targeting IL-17A comprises at least one CDR from an antibody light chain variable region, and the VL comprises an amino acid sequence as set forth in SEQ ID NO: 20.

In certain embodiments, the antigen binding protein targeting IL-17A comprises LCDR3, and the LCDR3 comprises an amino acid sequence as set forth in SEQ ID NO: 17.

In certain embodiments, the antigen binding protein targeting IL-17A comprises LCDR2, and the LCDR2 comprises an amino acid sequence as set forth in SEQ ID NO: 18 (KVS).

In certain embodiments, the antigen binding protein targeting IL-17A comprises LCDR1, and the LCDR1 comprises an amino acid sequence as set forth in SEQ ID NO: 19.

In certain embodiments, the antigen binding protein targeting IL-17A comprises VL, and the VL comprises an amino acid sequence as set forth in SEQ ID NO: 20.

In certain embodiments, the antigen binding protein targeting IL-17A comprises a light chain constant region.

In certain embodiments, the light chain constant region is derived from Igκ.

In certain embodiments, the heavy chain constant region of the antigen binding protein targeting IL-17A comprises an amino acid sequence as set forth in SEQ ID NO: 21.

In certain embodiments, the IL-36R is human IL-36R.

In certain embodiments, the antigen binding protein targeting IL-36R comprises at least one CDR from an antibody heavy chain variable region VH, and the VH comprises an amino acid sequence as set forth in SEQ ID NO: 4 or SEQ ID NO: 30.

In certain embodiments, the antigen binding protein targeting IL-36R comprises HCDR3, and the HCDR3 comprises an amino acid sequence as set forth in SEQ ID NO: 1 or SEQ ID NO: 27.

In certain embodiments, the antigen binding protein targeting IL-36R comprises HCDR2, and the HCDR2 comprises an amino acid sequence as set forth in SEQ ID NO: 2 or SEQ ID NO: 28.

In certain embodiments, the antigen binding protein targeting IL-36R comprises HCDR1, and the HCDR1 comprises an amino acid sequence as set forth in SEQ ID NO: 3 or SEQ ID NO: 29.

In certain embodiments, the antigen binding protein targeting IL-36R comprises VH, and the VH comprises an amino acid sequence as set forth in SEQ ID NO: 4 or SEQ ID NO: 30.

In certain embodiments, the antigen binding protein targeting IL-36R comprises an antibody heavy chain constant region.

In certain embodiments, the antibody heavy chain constant region is derived from a heavy chain constant region of IgG.

In certain embodiments, the antibody heavy chain constant region is derived from a heavy chain constant region of IgG1.

In certain embodiments, the antibody heavy chain constant region of the antigen binding protein targeting IL-36R comprises an amino acid sequence as set forth in SEQ ID NO: 9 or SEQ ID NO: 35.

In certain embodiments, the antigen binding protein targeting IL-36R comprises at least one CDR from an antibody light chain variable region, and the VL comprises an amino acid sequence as set forth in SEQ ID NO: 8 or SEQ ID NO: 34.

In certain embodiments, the antigen binding protein targeting IL-36R comprises LCDR3, and the LCDR3 comprises an amino acid sequence as set forth in SEQ ID NO: 5 or SEQ ID NO: 31.

In certain embodiments, the antigen binding protein targeting IL-36R comprises LCDR2, and the LCDR2 comprises an amino acid sequence as set forth in SEQ ID NO: 6 (STS) or SEQ ID NO: 32 (AAS).

In certain embodiments, the antigen binding protein targeting IL-36R comprises LCDR1, and the LCDR1 comprises an amino acid sequence as set forth in SEQ ID NO: 7 or SEQ ID NO: 33.

In certain embodiments, the antigen binding protein targeting IL-36R comprises VL, and the VL comprises an amino acid sequence as set forth in SEQ ID NO: 8 or SEQ ID NO: 34.

In certain embodiments, the antigen binding protein targeting IL-36R comprises a light chain constant region.

In certain embodiments, the light chain constant region is derived from Igκ.

In certain embodiments, the heavy chain constant region of the antigen binding protein targeting IL-36R comprises an amino acid sequence as set forth in SEQ ID NO: 9 or SEQ ID NO: 35.

In certain embodiments, the systemic lupus erythematosus comprises systemic lupus erythematosus nephritis.

In another aspect, the present application further provides the use of an isolated antigen binding protein in the preparation of a drug, wherein the isolated antigen binding protein comprises a first binding domain targeting IL-17A, and a second binding domain targeting IL-36R, and the drug is used to prevent and/or treat systemic lupus erythematosus.

In certain embodiments, the isolated antigen binding protein has one or more of the following properties:
(1) binding to IL-36R with high affinity;
(2) binding to IL-1Rrp2 with high affinity;
(3) blocking the binding of IL-36α, IL-36β, and IL-36γ to IL-36R; and
(4) blocking IL-36α-, IL-36β-, and IL-36γ-mediated cytokine secretion.

In certain embodiments, the isolated antigen binding protein is selected from a monoclonal antibody, a chimeric antibody, a humanized antibody, or a combination thereof.

In certain embodiments, the first binding domain of the isolated antigen binding protein comprises at least one CDR from an antibody heavy chain variable region VH, and the VH comprises an amino acid sequence as set forth in SEQ ID NO: 16.

In certain embodiments, the first binding domain of the isolated antigen binding protein comprises HCDR3, and the HCDR3 comprises an amino acid sequence as set forth in SEQ ID NO: 13.

In certain embodiments, the first binding domain of the isolated antigen binding protein comprises HCDR2, and the HCDR2 comprises an amino acid sequence as set forth in SEQ ID NO: 14.

In certain embodiments, the first binding domain of the isolated antigen binding protein comprises HCDR1, and the HCDR1 comprises an amino acid sequence as set forth in SEQ ID NO: 15.

In certain embodiments, the first binding domain of the isolated antigen binding protein comprises VH, and the VH comprises an amino acid sequence as set forth in SEQ ID NO: 16.

In certain embodiments, the first binding domain of the isolated antigen binding protein comprises at least one CDR from a light chain variable region VL, and the VL comprises an amino acid sequence as set forth in SEQ ID NO: 20.

In certain embodiments, the first binding domain of the isolated antigen binding protein comprises LCDR3, and the LCDR3 comprises an amino acid sequence as set forth in SEQ ID NO: 17.

In certain embodiments, the first binding domain of the isolated antigen binding protein comprises LCDR2, and the LCDR2 comprises an amino acid sequence as set forth in SEQ ID NO: 18 (KVS).

In certain embodiments, the first binding domain of the isolated antigen binding protein comprises LCDR1, and the LCDR1 comprises an amino acid sequence as set forth in SEQ ID NO: 19.

In certain embodiments, the first binding domain of the isolated antigen binding protein comprises VL, and the VL comprises an amino acid sequence as set forth in SEQ ID NO: 20.

In certain embodiments, the isolated antigen binding protein comprises a heavy chain constant region.

In certain embodiments, the isolated antigen binding protein comprises a light chain constant region.

In certain embodiments, the heavy chain constant region is derived from human IgG1 or human IgG4.

In certain embodiments, the light chain constant region is derived from human IgG1 or human IgG4.

In certain embodiments, the heavy chain constant region comprises an amino acid sequence as set forth in SEQ ID NO: 21.

In certain embodiments, the light chain constant region comprises an amino acid sequence as set forth in SEQ ID NO: 10.

In certain embodiments, the second binding domain of the isolated antigen binding protein comprises at least one CDR from an antibody heavy chain variable region VH, and the VH comprises an amino acid sequence as set forth in SEQ ID NO: 4 or SEQ ID NO: 30.

In certain embodiments, the second binding domain of the isolated antigen binding protein comprises HCDR3, and the HCDR3 comprises an amino acid sequence as set forth in SEQ ID NO: 1 or SEQ ID NO: 27.

In certain embodiments, the second binding domain of the isolated antigen binding protein comprises HCDR2, and the HCDR2 comprises an amino acid sequence as set forth in SEQ ID NO: 2 or SEQ ID NO: 28.

**In** certain embodiments, the second binding domain of the isolated antigen binding protein comprises HCDR1, and the HCDR1 comprises an amino acid sequence as set forth in SEQ ID NO: 3 or SEQ ID NO: 29.

In certain embodiments, the second binding domain of the isolated antigen binding protein comprises VH, and the VH comprises an amino acid sequence as set forth in SEQ ID NO: 4 or SEQ ID NO: 30.

In certain embodiments, the second binding domain of the isolated antigen binding protein comprises at least one CDR from an antibody light chain variable region VL, and the VL comprises an amino acid sequence as set forth in SEQ ID NO: 8 or SEQ ID NO: 34.

In certain embodiments, the second binding domain of the isolated antigen binding protein comprises LCDR3, and the LCDR3 comprises an amino acid sequence as set forth in SEQ ID NO: 5 or SEQ ID NO: 31.

In certain embodiments, the second binding domain of the isolated antigen binding protein comprises LCDR2, and the LCDR2 comprises an amino acid sequence as set forth in SEQ ID NO: 6 (STS) or SEQ ID NO: 32 (AAS).

In certain embodiments, the second binding domain of the isolated antigen binding protein comprises LCDR1, and the LCDR1 comprises an amino acid sequence as set forth in SEQ ID NO: 7 or SEQ ID NO: 33.

In certain embodiments, the second binding domain of the isolated antigen binding protein comprises VL, and the VL comprises an amino acid sequence as set forth in SEQ ID NO: 8 or SEQ ID NO: 34.

In certain embodiments, the second binding domain of the isolated antigen binding protein comprises VH and VL, and the VH and VL are linked via a linking peptide.

In certain embodiments, the amino acid sequence of the linking peptide is (G4S)n, wherein n is a positive integer.

In certain embodiments, the second binding domain of the isolated antigen binding protein is scFv.

In certain embodiments, the scFv of the second binding domain of the isolated antigen binding protein comprises an amino acid sequence as set forth in SEQ ID NO: 24.

In certain embodiments, the isolated antigen binding protein comprises: (a) a first binding domain of an anti-IL-17A antibody; and (b) a second binding domain linked to the anti-IL-17A antibody.

In certain embodiments, the second binding domain is a single-chain variable region (scFv) of an anti-IL-36R antibody.

In certain embodiments, the second binding domain is directly or indirectly linked to the first binding domain.

In certain embodiments, the second binding domain is linked to the first binding domain via a linker.

In certain embodiments, the anti-IL-17A antibody and the ScFv of the anti-IL-36R antibody are linked via a linker sequence.

In certain embodiments, the isolated antigen binding protein is a homodimer.

In certain embodiments, the isolated antigen binding protein is a tetravalent antibody.

In certain embodiments, the isolated antigen binding protein is a bispecific antibody.

In certain embodiments, the bispecific antibody is a dual-chain antibody or a single-chain antibody.

In certain embodiments, the bispecific antibody has a structure represented by formula I from N-terminus to C-terminus: 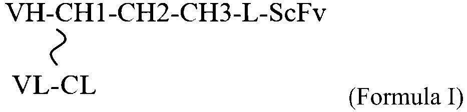 wherein,
VH represents the heavy chain variable region of the anti-IL-17A antibody;
VL represents the light chain variable region of the anti-IL-17A antibody;
CH1, CH2 and CH3 represent the heavy chain constant regions CH1, CH2 and CH3 of the anti-IL-17A antibody, respectively;
CL represents the light chain constant region of the anti-IL-17A antibody;
ScFv represents the ScFv of the anti-IL-36R antibody;
L is a linker element;
"~" represents a disulfide bond;
"-" represents a peptide bond;
wherein, the bispecific antibody has an activity of binding to IL-17A and IL-36R simultaneously.

In certain embodiments, the bispecific antibody is formed by fusion of the anti-IL-17A antibody and the scFv of the anti-IL-36R antibody, and has two pairs of peptide chains that are symmetrical to each other; each pair of peptide chains comprises a light chain L chain and a heavy chain H chain; all the peptide chains are linked via disulfide bonds, and any pair of peptide chains has a structure of the H chain and the L chain represented by formula I from N-terminus to C-terminus.

In certain embodiments, the bispecific antibody is a homodimer of the peptide chain of the structure represented by formula I.

In certain embodiments, the linker element is a linking peptide.

In certain embodiments, the amino acid sequence of the linking peptide is (G4S)n, wherein n is a positive integer. The H chain of the bispecific antibody has an amino acid sequence as set forth in SEQ ID NO: 25; and the L chain of the bispecific antibody has an amino acid sequence as set forth in SEQ ID NO: 23.

In certain embodiments, the isolated antigen binding protein further comprises a detectable marker, a targeting marker, a drug, a toxin, a cytokine, a radionuclide, or an enzyme.

In certain embodiments, the systemic lupus erythematosus comprises lupus erythematosus nephritis.

In certain embodiments, the drug is capable of alleviating the elevated urine protein level caused by systemic lupus erythematosus.

In certain embodiments, the drug is capable of alleviating renal pathological damage caused by systemic lupus erythematosus.

In certain embodiments, the drug is capable of alleviating and/or inhibiting renal fibrosis caused by systemic lupus erythematosus.

In another aspect, the present application further provides a method for preventing and/or treating systemic lupus erythematosus, comprising administering a drug to a subject in need thereof, wherein the drug is capable of targeting IL-17A and IL-36R.

In certain embodiments, the systemic lupus erythematosus comprises lupus erythematosus nephritis.

In another aspect, the present application further provides a method for alleviating elevated urine protein level caused by systemic lupus erythematosus, comprising administering a drug to a subject in need thereof, wherein the drug is capable of targeting IL-17A and IL-36R.

In another aspect, the present application further provides a method for alleviating renal pathological damage caused by systemic lupus erythematosus, comprising administering a drug to a subject in need thereof, wherein the drug is capable of targeting IL-17A and IL-36R.

In another aspect, the present application further provides a method for alleviating and/or inhibiting renal fibrosis caused by systemic lupus erythematosus, comprising administering a drug to a subject in need thereof, wherein the drug is capable of targeting IL-17A and IL-36R.

In certain embodiments, the IL-17A is human IL-17A.

In certain embodiments, the IL-36R is human IL-36R.

In certain embodiments, the drug comprises an antigen binding protein targeting IL-17A, and an antigen binding protein targeting IL-36R.

In certain embodiments, the antigen binding protein targeting IL-17A is capable of blocking the related effects of IL-17A and IL-17R.

In certain embodiments, the antigen binding protein targeting IL-17A comprises at least one CDR from an antibody heavy chain variable region VH, and the VH comprises an amino acid sequence as set forth in SEQ ID NO: 16.

In certain embodiments, the antigen binding protein targeting IL-17A comprises HCDR3, and the HCDR3 comprises an amino acid sequence as set forth in SEQ ID NO: 13.

In certain embodiments, the antigen binding protein targeting IL-17A comprises HCDR2, and the HCDR2 comprises an amino acid sequence as set forth in SEQ ID NO: 14.

In certain embodiments, the antigen binding protein targeting IL-17A comprises HCDR1, and the HCDR1 comprises an amino acid sequence as set forth in SEQ ID NO: 15.

In certain embodiments, the antigen binding protein targeting IL-17A comprises VH, and the VH comprises an amino acid sequence as set forth in SEQ ID NO: 16.

In certain embodiments, the antigen binding protein targeting IL-17A comprises an antibody heavy chain constant region.

In certain embodiments, the antibody heavy chain constant region is derived from a heavy chain constant region of IgG.

In certain embodiments, the antibody heavy chain constant region is derived from a heavy chain constant region of IgG1.

In certain embodiments, the antibody heavy chain constant region comprises an amino acid sequence as set forth in SEQ ID NO: 21.

In certain embodiments, the antigen binding protein targeting IL-17A comprises at least one CDR from an antibody light chain variable region, and the VL comprises an amino acid sequence as set forth in SEQ ID NO: 20.

In certain embodiments, the antigen binding protein targeting IL-17A comprises LCDR3, and the LCDR3 comprises an amino acid sequence as set forth in SEQ ID NO: 17.

In certain embodiments, the antigen binding protein targeting IL-17A comprises LCDR2, and the LCDR2 comprises an amino acid sequence as set forth in SEQ ID NO: 18 (KVS).

In certain embodiments, the antigen binding protein targeting IL-17A comprises LCDR1, and the LCDR1 comprises an amino acid sequence as set forth in SEQ ID NO: 19.

In certain embodiments, the antigen binding protein targeting IL-17A comprises VL, and the VL comprises an amino acid sequence as set forth in SEQ ID NO: 20.

In certain embodiments, the antigen binding protein targeting IL-17A comprises a light chain constant region.

In certain embodiments, the light chain constant region is derived from Igκ.

In certain embodiments, the light chain constant region comprises an amino acid sequence as set forth in SEQ ID NO: 10.

In certain embodiments, the antigen binding protein targeting IL-36R comprises at least one CDR from an antibody heavy chain variable region VH, and the VH comprises an amino acid sequence as set forth in SEQ ID NO: 4 or SEQ ID NO: 30.

In certain embodiments, the antigen binding protein targeting IL-36R comprises HCDR3, and the HCDR3 comprises an amino acid sequence as set forth in SEQ ID NO: 1 or SEQ ID NO: 27.

In certain embodiments, the antigen binding protein targeting IL-36R comprises HCDR2, and the HCDR2 comprises an amino acid sequence as set forth in SEQ ID NO: 2 or SEQ ID NO: 28.

In certain embodiments, the antigen binding protein targeting IL-36R comprises HCDR1, and the HCDR1 comprises an amino acid sequence as set forth in SEQ ID NO: 3 or SEQ ID NO: 29.

In certain embodiments, the antigen binding protein targeting IL-36R comprises VH, and the VH comprises an amino acid sequence as set forth in SEQ ID NO: 4 or SEQ ID NO: 30.

In certain embodiments, the antigen binding protein targeting IL-36R comprises an antibody heavy chain constant region.

In certain embodiments, the antibody heavy chain constant region is derived from a heavy chain constant region of IgG.

In certain embodiments, the antibody heavy chain constant region is derived from a heavy chain constant region of IgG1.

In certain embodiments, the antibody heavy chain constant region comprises an amino acid sequence as set forth in SEQ ID NO: 9 or SEQ ID NO: 35.

In certain embodiments, the antigen binding protein targeting IL-36R comprises at least one CDR from an antibody light chain variable region, and the VL comprises an amino acid sequence as set forth in SEQ ID NO: 8 or SEQ ID NO: 34.

In certain embodiments, the antigen binding protein targeting IL-36R comprises LCDR3, and the LCDR3 comprises an amino acid sequence as set forth in SEQ ID NO: 5 or SEQ ID NO: 31.

In certain embodiments, the antigen binding protein targeting IL-36R comprises LCDR2, and the LCDR2 comprises an amino acid sequence as set forth in SEQ ID NO: 6 (STS) or SEQ ID NO: 32 (AAS).

In certain embodiments, the antigen binding protein targeting IL-36R comprises LCDR1, and the LCDR1 comprises an amino acid sequence as set forth in SEQ ID NO: 7 or SEQ ID NO: 33.

In certain embodiments, the antigen binding protein targeting IL-36R comprises VL, and the VL comprises an amino acid sequence as set forth in SEQ ID NO: 8 or SEQ ID NO: 34.

In certain embodiments, the antigen binding protein targeting IL-36R comprises a light chain constant region.

In certain embodiments, the light chain constant region is derived from Igκ.

In certain embodiments, the light chain constant region comprises an amino acid sequence as set forth in SEQ ID NO: 10 or SEQ ID NO: 36.

In certain embodiments, the drug comprises an isolated antigen binding protein, and the isolated antigen binding protein comprises a first binding domain targeting IL-17A, and a second binding domain targeting IL-36R.

In certain embodiments, the isolated antigen binding protein has one or more of the following properties:
(1) binding to IL-36R with high affinity;
(2) binding to IL-1Rrp2 with high affinity;
(3) blocking the binding of IL-36α, IL-36β, and IL-36γ to IL-36R; and
(4) blocking IL-36α-, IL-36β-, and IL-36γ-mediated cytokine secretion.

In certain embodiments, the isolated antigen binding protein is selected from a monoclonal antibody, a chimeric antibody, a humanized antibody, or a combination thereof.

In certain embodiments, the first binding domain of the isolated antigen binding protein comprises at least one CDR from an antibody heavy chain variable region VH, and the VH comprises an amino acid sequence as set forth in SEQ ID NO: 16.

In certain embodiments, the first binding domain of the isolated antigen binding protein comprises HCDR3, and the HCDR3 comprises an amino acid sequence as set forth in SEQ ID NO: 13.

In certain embodiments, the first binding domain of the isolated antigen binding protein comprises HCDR2, and the HCDR2 comprises an amino acid sequence as set forth in SEQ ID NO: 14.

In certain embodiments, the first binding domain of the isolated antigen binding protein comprises HCDR1, and the HCDR1 comprises an amino acid sequence as set forth in SEQ ID NO: 15.

In certain embodiments, the first binding domain of the isolated antigen binding protein comprises VH, and the VH comprises an amino acid sequence as set forth in SEQ ID NO: 16.

In certain embodiments, the first binding domain of the isolated antigen binding protein comprises at least one CDR from a light chain variable region VL, and the VL comprises an amino acid sequence as set forth in SEQ ID NO: 20.

In certain embodiments, the first binding domain of the isolated antigen binding protein comprises LCDR3, and the LCDR3 comprises an amino acid sequence as set forth in SEQ ID NO: 17.

In certain embodiments, the first binding domain of the isolated antigen binding protein comprises LCDR2, and the LCDR2 comprises an amino acid sequence as set forth in SEQ ID NO: 18 (KVS).

In certain embodiments, the first binding domain of the isolated antigen binding protein comprises LCDR1, and the LCDR1 comprises an amino acid sequence as set forth in SEQ ID NO: 19.

In certain embodiments, the first binding domain of the isolated antigen binding protein comprises VL, and the VL comprises an amino acid sequence as set forth in SEQ ID NO: 20.

In certain embodiments, the isolated antigen binding protein comprises a heavy chain constant region.

In certain embodiments, the isolated antigen binding protein comprises a light chain constant region.

In certain embodiments, the heavy chain constant region is derived from human IgG1 or human IgG4.

In certain embodiments, the light chain constant region is derived from human IgG1 or human IgG4.

In certain embodiments, the second binding domain of the isolated antigen binding protein comprises at least one CDR from an antibody heavy chain variable region VH, and the VH comprises an amino acid sequence as set forth in SEQ ID NO: 4 or SEQ ID NO: 30.

In certain embodiments, the second binding domain of the isolated antigen binding protein comprises HCDR3, and the HCDR3 comprises an amino acid sequence as set forth in SEQ ID NO: 1 or SEQ ID NO: 27.

In certain embodiments, the second binding domain of the isolated antigen binding protein comprises HCDR2, and the HCDR2 comprises an amino acid sequence as set forth in SEQ ID NO: 2 or SEQ ID NO: 28.

**In** certain embodiments, the second binding domain of the isolated antigen binding protein comprises HCDR1, and the HCDR1 comprises an amino acid sequence as set forth in SEQ ID NO: 3 or SEQ ID NO: 29.

In certain embodiments, the second binding domain of the isolated antigen binding protein comprises VH, and the VH comprises an amino acid sequence as set forth in SEQ ID NO: 4 or SEQ ID NO: 30.

In certain embodiments, the second binding domain of the isolated antigen binding protein comprises at least one CDR from an antibody light chain variable region VL, and the VL comprises an amino acid sequence as set forth in SEQ ID NO: 8 or SEQ ID NO: 34.

In certain embodiments, the second binding domain of the isolated antigen binding protein comprises LCDR3, and the LCDR3 comprises an amino acid sequence as set forth in SEQ ID NO: 5 or SEQ ID NO: 31.

In certain embodiments, the second binding domain of the isolated antigen binding protein comprises LCDR2, and the LCDR2 comprises an amino acid sequence as set forth in SEQ ID NO: 6 (STS) or SEQ ID NO: 32 (AAS).

In certain embodiments, the second binding domain of the isolated antigen binding protein comprises LCDR1, and the LCDR1 comprises an amino acid sequence as set forth in SEQ ID NO: 7 or SEQ ID NO: 33.

In certain embodiments, the second binding domain of the isolated antigen binding protein comprises VL, and the VL comprises an amino acid sequence as set forth in SEQ ID NO: 8 or SEQ ID NO: 34.

In certain embodiments, the second binding domain of the isolated antigen binding protein comprises VH and VL, and the VH and VL are linked via a linking peptide.

In certain embodiments, the amino acid sequence of the linking peptide is (G4S)n, wherein n is a positive integer.

In certain embodiments, the second binding domain of the isolated antigen binding protein is scFv.

In certain embodiments, the scFv of the second binding domain of the isolated antigen binding protein comprises an amino acid sequence as set forth in SEQ ID NO: 24.

In certain embodiments, the isolated antigen binding protein comprises: (a) a first binding domain of an anti-IL-17A antibody; and (b) a second binding domain linked to the anti-IL-17A antibody.

In certain embodiments, the second binding domain is a single-chain variable region (scFv) of an anti-IL-36R antibody.

In certain embodiments, the second binding domain is directly or indirectly linked to the first binding domain.

In certain embodiments, the second binding domain is linked to the first binding domain via a linker.

In certain embodiments, the anti-IL-17A antibody and the scFv of the anti-IL-36R antibody are linked via a linker sequence.

In certain embodiments, the isolated antigen binding protein is a homodimer.

In certain embodiments, the isolated antigen binding protein is a tetravalent antibody.

In certain embodiments, the isolated antigen binding protein is a bispecific antibody.

In certain embodiments, the bispecific antibody is a dual-chain antibody or a single-chain antibody.

In certain embodiments, the bispecific antibody has a structure represented by formula I from N-terminus to C-terminus: 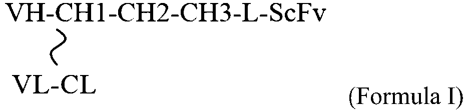 wherein,
VH represents the heavy chain variable region of the anti-IL-17A antibody;
VL represents the light chain variable region of the anti-IL-17A antibody;
CH1, CH2 and CH3 represent the heavy chain constant regions CH1, CH2 and CH3 of the anti-IL-17A antibody, respectively;
CL represents the light chain constant region of the anti-IL-17A antibody;
ScFv represents the scFv of the anti-IL-36R antibody;
L is a linker element;
"~" represents a disulfide bond;
"-" represents a peptide bond;
wherein, the bispecific antibody has an activity of binding to IL-17A and IL-36R simultaneously.

In certain embodiments, the bispecific antibody is formed by fusion of the anti-IL-17A antibody and the ScFv of the anti-IL-36R antibody, and has two pairs of peptide chains that are symmetrical to each other; each pair of peptide chains comprises a light chain L chain and a heavy chain H chain; all the peptide chains are linked via disulfide bonds, and any pair of peptide chains has a structure of the H chain and the L chain represented by formula I from N-terminus to C-terminus.

In certain embodiments, the bispecific antibody is a homodimer of the peptide chain of the structure represented by formula I.

In certain embodiments, the linker element is a linking peptide.

In certain embodiments, the amino acid sequence of the linking peptide is (G4S)n, wherein n is a positive integer.

In certain embodiments, the H chain of the bispecific antibody has an amino acid sequence as set forth in SEQ ID NO: 25; and the L chain of the bispecific antibody has an amino acid sequence as set forth in SEQ ID NO: 23.

In certain embodiments, the isolated antigen binding protein further comprises a detectable marker, a targeting marker, a drug, a toxin, a cytokine, a radionuclide, or an enzyme.

In certain embodiments, the drug further comprises an optional pharmaceutically acceptable carrier.

In certain embodiments, the drug comprises a nucleic acid molecule encoding the antigen binding protein.

Those skilled in the art can easily discern other aspects and advantages of the present application from the following detailed description. Only exemplary embodiments of the present application are shown and described in the following detailed description. As those skilled in the art will recognize, the content of the present application enables those skilled in the art to make changes to the disclosed specific embodiments without departing from the spirit and scope of the invention involved in the present application. Accordingly, the drawings and descriptions in the specification of the present application are illustrative only and not restrictive.

### BRIEF DESCRIPTION OF DRAWINGS

The specific features of the invention to which the present application relates are set forth in the appended claims. The features and advantages of the invention involved in the present application can be better understood by referring to the exemplary embodiments and figures described in detail below. The accompanying drawings are briefly described as follows:
FIG. 1 shows a schematic diagram of the structure of the HB0043 bispecific antibody.
FIG. 2 shows the inhibitory effect of the antibody of the present application on IL-6 under the stimulation of IL-17A and IL-36α.
FIG. 3 shows the inhibitory effect of the antibody of the present application on IL-6 under the stimulation of IL-17A and IL-36β.
FIG. 4 shows the inhibitory effect of the antibody of the present application on IL-6 under the stimulation of IL-17A and IL-36γ.
FIG. 5 shows the inhibitory effect of the antibody of the present application on IL-8 under the stimulation of IL-17A and IL-36α.
FIG. 6 shows the inhibitory effect of the antibody of the present application on IL-8 under the stimulation of IL-17A and IL-36β.
FIG. 7 shows the inhibitory effect of the antibody of the present application on IL-8 under the stimulation of IL-17A and IL-36γ.
FIG. 8 shows the kidney coefficient of the UUO side of mice (kidney coefficient = kidney mass on the surgical side/mouse body weight).
FIG. 9 shows the detection of serum creatinine content in mice.
FIG. 10 shows the detection of serum BUN content in mice.
FIG. 11 shows kidney PAS staining of the UUO side of mice.
FIG. 12 shows the quantitative results of glomerular fibrosis (*** P < 0.001, vs. normal control; ## P < 0.01, ### P < 0.001, vs. 900543).
FIG. 13 shows the quantitative results of tubulointerstitial fibrosis.
FIG. 14 shows kidney Masson staining of the UUO side of mice.
FIG. 15 shows the results of kidney FN immunohistochemistry of the UUO side of mice.
FIG. 16 shows the changes in body weight of NZB/NZW F1 mice after combined administration of IL-17A antibody and IL-36R antibody.
FIG. 17 shows the detection of urine protein levels in NZB/NZW F1 mice after combined administration of IL-17A antibody and IL-36R antibody.
FIG. 18 shows the evaluation of renal pathological damage in NZB/NZW F1 mice after combined administration of IL-17A antibody and IL-36R antibody.
FIG. 19 shows the detection of renal fibrosis in NZB/NZW F1 mice after combined administration of IL-17A antibody and IL-36R antibody.
FIG. 20 shows the detection of mortality of MRL/lpr mice after combined administration of IL-17A antibody and IL-36R antibody.
FIG. 21 shows the detection of urine protein levels in MRL/lpr mice after combined administration of IL-17A antibody and IL-36R antibody.
FIG. 22 shows the detection of renal fibrosis in MRL/lpr mice after combined administration of IL-17A antibody and IL-36R antibody.

### DETAILED DESCRIPTION

The embodiments of the invention of the present application will be illustrated below with specific examples. Those skilled in the art may easily understand other advantages and effects of the invention of the present application from the disclosure of the specification.

### DEFINITION OF TERMS

In the present application, the term "isolated" typically refers to something obtained through artificial means from the natural state. If an "isolated" substance or component occurs in nature, it may be that the natural environment in which the substance or component is has changed, or that the substance has been isolated from the natural environment, or both. For example, a certain unisolated polynucleotide or polypeptide naturally exists in a living animal, and the same polynucleotide or polypeptide with high purity isolated from the natural state is called "isolated". The term "isolated" does not exclude the presence of artificial or synthetic substances, nor does it exclude the presence of other impure substances that do not affect the activity of the substance isolated.

In the present application, the term "isolated antigen binding protein" typically refers to a protein with antigen binding capability that has been separated from its naturally occurring state. The "isolated antigen binding protein" may include a portion that binds to an antigen and, optionally, a framework or framework portion that allows the antigen binding portion to adopt a conformation that promotes the antigen binding portion to bind to the antigen. The antigen binding protein may include, for example, antibody-derived protein framework regions (FRs) or alternative protein framework regions or artificial framework regions with grafted CDRs or CDR derivatives. Such frameworks include, but are not limited to, antibody-derived framework regions comprising mutations introduced, for example, to stabilize the three-dimensional structure of the antigen binding protein, as well as fully synthetic framework regions comprising, for example, biocompatible polymers. See, for example, Korndorfer et al., 2003, Proteins: Structure, Function, and Bioinformatics, 53(1):121-129 (2003); Roque et al., Biotechnol. Prog. 20:639-654 (2004). Examples of antigen binding proteins include, but are not limited to: human antibodies, humanized antibodies; chimeric antibodies; recombinant antibodies; single chain antibodies; bifunctional antibodies; trifunctional antibodies; tetrafunctional antibodies; Fab, Fab', Fv fragments, F(ab')₂, F(ab)₂, scFv, di-scFv, dAb, IgD antibody; IgE antibody; IgM antibody; IgG1 antibody; IgG2 antibody; IgG3 antibody; or IgG4 antibody and fragments thereof.

In the present application, the term "antibody" typically refers to an immunoglobulin molecule, which is typically a tetrapeptide chain structure composed of two identical heavy chains and two identical light chains connected by inter-chain disulfide bonds. Since the amino acid composition and arrangement sequence of the heavy chain constant region of an immunoglobulin are different, the antigenicity of the immunoglobulin is also different. Accordingly, immunoglobulins can be divided into five categories, or different isotypes of immunoglobulins, namely IgM, IgD, IgG, IgA and IgE. The heavy chain constant regions corresponding to different categories of immunoglobulins are called α, δ, ε, γ and µ, respectively. IgG represents the most important class of immunoglobulins. Due to differences in chemical structure and biological functions, IgG can be divided into 4 subclasses: IgG1, IgG2, IgG3 and IgG4. Light chains are divided into kappa or lambda chains by differences in constant regions. The subunit structures and three-dimensional configurations of different categories of immunoglobulins are well known to those in the art. In the present application, the term encompasses monoclonal antibodies, polyclonal antibodies, murine antibodies, humanized antibodies, chimeric antibodies, bispecific antibodies, multispecific antibodies, and the like.

In the present application, the term "CDR", also known as "complementarity determining region", typically refers to a region in an antibody variable domain whose sequence is highly variable and/or forms a structurally defined loop. Typically, the antibody includes six CDRs, three (HCDR1, HCDR2, HCDR3) in VH, and three (LCDR1, LCDR2, LCDR3) in VL. In certain embodiments, naturally occurring camelid antibodies consisting solely of heavy chains function normally and stably in the absence of light chains. See, e.g., Hamers-Casterman et al., Nature 363:446-448 (1993); Sheriff et al, Nature Struct. Biol. 3:733-736 (1996). An antibody CDR may be determined by a variety of encoding systems, e.g., CCG, Kabat, Chothia, IMGT, and Kabat/Chothia considered comprehensively. These encoding systems are known in the art, and specific reference may be made to, for example, http://www.bioinf.org.uk/abs/index.html#kabatnum. For example, the amino acid sequence of the antigen binding protein can be numbered according to the IMGT numbering scheme (IMGT, the international ImMunoGeneTics information system@imgt.cines.fr; http://imgt.cines.fr; Lefranc et al., 1999, Nucleic Acids Res. 27: 209-212; Ruiz et al., 2000 Nucleic Acids Res. 28: 219-221; Lefranc et al., 2001, Nucleic Acids Res. 29: 207-209; Lefranc et al., 2003, Nucleic Acids Res. 31: 307-310; Lefranc et al., 2005, DevComp Immunol 29: 185-203). For example, the CDRs of the antigen binding protein can be determined according to the Kabat numbering system (see, e.g., Kabat EA & Wu TT (1971) Ann NY Acad Sci 190:382-391 and Kabat EA et al., (1991) Sequences of Proteins of Immunological Interest, Fifth Edition, U.S. Department of Health and Human Services, NIH Publication No. 91-3242).

In the present application, the term "FR" typically refers to the more highly conserved portion of the variable domain of an antibody, which is called the framework region. Typically, the variable domains of natural heavy chains and light chains each contains four FR regions, namely, four (H-FR1, H-FR2, H-FR3 and H-FR4) in VH, and four (L-FR1, L-FR2, L-FR3 and L-FR4) in VL.

In the present application, the term "variable domain" and "variable region" are interchangeably used with each other, and typically refer to a part of antibody heavy chains/or light chains. The variable domains of a heavy chain and a light chain may be referred to as "V_{H}" and "V_{L}" (or "VH" and "VL"), respectively. These domains are typically the most variable portion of an antibody (relative to other antibodies of the same type) and contain the antigen binding site.

In the present application, the term "variable" typically refers to the fact that certain segments of the variable domain may differ greatly in sequence between antibodies. A variable domain mediates antigen binding and determines the specificity of a specific antibody for its specific antigen. However, the variability is not evenly distributed throughout the variable domain. It is usually concentrated in three segments called hypervariable regions (CDRs or HVRs) in both the light chain and heavy chain variable domains. The more highly conserved portions in the variable domains are called framework regions (FRs). The variable domains of native heavy and light chains each comprise four FR regions, most of which adopt a β-sheet conformation and are linked by three CDRs. These CDRs form loop structures and, in some cases, become part of the β-sheet structure. The CDRs in each chain are held together in close proximity by the FR regions, and together with the CDRs from the other chain contribute to the formation of the antigen binding site of the antibody (see Kabat et al, Sequences of Immunological Interest, Fifth Edition, National Institute of Health, Bethesda, Md. (1991)).

In the present application, the term "antigen binding fragment" typically refers to a Fab fragment, a Fab' fragment, a F(ab')2 fragment, or a single Fv fragment having antigen binding activity. Fv antibodies contain the heavy chain variable region and the light chain variable region of the antibody, but no constant region, and are the smallest antibody fragments with all antigen binding sites. Typically, Fv antibodies further contain a polypeptide linker between the VH and VL domains, and are capable of forming the structure required for antigen binding.

In the present application, the term "Fab" typically refers to the antigen binding fragment of an antibody. As described above, intact antibodies can be digested using papain. The antibody is digested by papain to produce two identical antigen binding fragments, namely the "Fab" fragment, and a residual "Fc" fragment (i.e., Fc region, ibid.). The Fab fragment can consist of an intact L chain and a variable region of a heavy chain along with the first constant region (CH1) of the H chain (VH).

In the present application, the term "Fab' fragment" typically refers to a monovalent antigen binding fragment of a human monoclonal antibody, which is slightly larger than the Fab fragment. For example, an Fab' fragment may include all light chains, all heavy chain variable regions, and all or part of the first and second constant regions of the heavy chains. For example, the Fab' fragment may also include part or all of the 220-330 amino acid residues of the heavy chain.

In the present application, the term "F(ab')2" typically refers to an antibody fragment produced by digesting an intact antibody with pepsin. The F(ab')2 fragment contains two Fab fragments held together by disulfide bonds and part of the hinge region. The F(ab')2 fragment has divalent antigen binding activity and is capable of cross-linking antigens.

In the present application, the term "Fv fragment" typically refers to a monovalent antigen binding fragment of a human monoclonal antibody, including all or part of the heavy chain variable region and the light chain variable region, and lacking the heavy chain constant region and the light chain constant region. A heavy chain variable region and a light chain variable region include, for example, CDRs. For example, the Fv fragment includes all or part of the amino terminal variable region of about 110 amino acids of the heavy chain and the light chain.

In the present application, the term "scFv" typically refers to a fusion protein comprising at least one antibody fragment including the variable region of the light chain and at least one antibody fragment including the variable region of the heavy chain, wherein the light chain and heavy chain variable regions are adjacent (e.g., via a synthetic linker such as a short flexible polypeptide linker) and can be expressed in the form of a single-chain polypeptide, and wherein the scFv retains the specificity of the intact antibody from which it is derived. Unless otherwise specified, as used in the present application, scFv can have the VL and VH variable regions in any order (e.g., relative to the N-terminus and C-terminus of the polypeptide), and scFv can include VL-linker-VH or can include VH-linker-VL.

In the present application, the term "dAb" typically refers to an antigen binding fragment having a VH domain, a VL domain, or a VH domain or a VL domain, see, for example, Ward et al. (Nature, 1989 Oct 12; 341 (6242): 544-6), Holt et al., Trends Biotechnol., 2003, 21 (11): 484-490; and see, for example, WO 06/030220, WO 06/003388, and other published patent applications of Domantis Ltd.

The present application includes not only intact antibodies, but also fragments of antibodies having immunological activity or fusion proteins formed by antibodies and other sequences. Therefore, the present application further includes fragments, derivatives and analogs of the antibodies.

In the present application, the term "monoclonal antibody" refers to an antibody secreted by a clone derived from a single cell source. Monoclonal antibodies are highly specific and are directed against a single antigen epitope. The cells may be eukaryotic, prokaryotic, or phage clonal cell lines.

In the present application, the term "chimeric antibody" refers to an antibody molecule that is formed by splicing the V region gene of a mouse antibody with the C region gene of a human antibody into a chimeric gene, which is then inserted into a vector and transfected into a host cell for expression. It not only retains the high specificity and affinity of the parent mouse antibody, but also enables its human Fc segment to effectively mediate biological effect functions.

In the present application, the term "humanized antibody" typically refers to a variable region modification form of an antibody, which has a CDR region derived from (or substantially derived from) a non-human antibody (for example, a mouse monoclonal antibody), and a FR region and a constant region substantially derived from a human antibody sequence; that is, the CDR region sequence of the mouse antibody is grafted onto different types of human germline antibody framework sequences. Because the CDR sequence is responsible for most of the antibody-antigen interaction, recombinant antibodies that mimic the properties of specific naturally occurring antibodies can be expressed by constructing an expression vector.

In the present application, the antibody can be monospecific, bispecific, or trispecific, or exhibit higher orders of multispecificity.

In the present application, the antibody of the present application further includes its conservative variants, which means that compared with the amino acid sequence of the antibody of the present application, up to 10, for example, up to 8, for example, up to 5, for example, up to 3 amino acids are replaced by amino acids with similar or close properties to form a polypeptide. These conservative variant peptides are preferably generated by amino acid substitution according to Table A.

**Table A**

| Original residues | Representative substitutions | For example, substituted with |
|---|---|---|
| Ala (A) | Vai; Leu; He | Vai |
| Arg (R) | Lys; Gin; Asn | Lvs |
| Asn (N) | Gin; His; Lys; Arg | Gin |
| Asp (D) | Glu | Glu |
| Cys (C) | Ser | Ser |
| Gin (Q) | Asn | Asn |
| Glu (E) | Asp | Asp |
| Gly (G) | Pro; Ala | Ala |
| His (H) | Asn; Gin; Lys; Arg | Arg |
| He (I) | Leu; Vai; Met; Ala; Phe | Leu |
| Leu (L) | He; Vai; Met; Ala; Phe | Ile |
| Lys (K) | Arg; Gin; Asn | Arg |
| Met (M) | Leu; Phe; He | Leu |
| Phe (F) | Leu; Vai; lie; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tvr (Y) | Trp; Phe; Thr; Ser | Phe |
| Vai (V) | He; Leu; Met; Phe; Ala | Leu |

In the present application, the term "IL-36R" typically refers to natural or recombinant IL-36R, and non-human homologs of IL-36R. For example, the IL-36R can be human IL-36R.

In the present application, the term "IL-17R" typically refers to natural or recombinant IL-17A, and non-human homologs of IL-17A. For example, the IL-17A can be human IL-17A.

In the present application, the term "nucleic acid molecule" typically refers to nucleotides of any length in isolated form, either deoxyribonucleotides or ribonucleotides, or analogs isolated from their natural environment or artificially synthesized.

In the present application, the term "pharmaceutically acceptable carrier" typically includes pharmaceutically acceptable carriers, excipients or stabilizers that are non-toxic at the doses and concentrations employed to the cells or mammals to which they are exposed. Physiologically acceptable carriers may include, for example, buffers, antioxidants, low molecular weight (less than about 10 residues) polypeptides, proteins, hydrophilic polymers, amino acids, monosaccharides, disaccharides and other carbohydrates, chelating agents, sugar alcohols, salt-forming counterions, such as sodium; and/or nonionic surfactants.

In the present application, the term "specific binding" or "specific" typically refers to a measurable and reproducible interaction, such as binding between a target and an antibody, that determines the presence of a target in the presence of a heterogeneous population of molecules, including biomolecules. For example, an antibody that specifically binds a target (which may be an epitope) may be an antibody that binds to that target with greater affinity, avidity, more readily, and/or for a greater duration than it binds to other targets. In some embodiments, the antibody specifically binds to an epitope on a protein, and the epitope is conserved among proteins in different species. In certain embodiments, specific binding includes, but does not require, exclusive binding.

In the present application, the term "subject" typically refers to a human or non-human animal including, but not limited to, a cat, dog, horse, pig, cow, sheep, rabbit, mouse, rat, or monkey.

In the present application, the proteins, polypeptides and/or amino acid sequences involved should also be understood to include at least the following: variants or homologs that have the same or similar functions as the protein or polypeptide.

In the present application, the variant may be, for example, a protein or polypeptide in which one or more amino acids are substituted, deleted or added in the amino acid sequence of the protein and/or the polypeptide (e.g., an antibody or fragment thereof that specifically binds to the CD73 protein). For example, the functional variants may include proteins or polypeptides with amino acid alterations through substitution, deletion and/or insertion of at least 1, e.g., 1-30, 1-20, 1-10, 1, 2, 3, 4 or 5 amino acids. The functional variant may substantially retain the biological properties of the protein or polypeptide prior to the alteration (e.g., substitution, deletion, or addition). For example, the functional variant may retain at least 60%, 70%, 80%, 90%, or 100% of the biological activity (e.g., antigen binding ability) of the protein or polypeptide prior to the alteration. For example, the substitution may be a conservative substitution.

In the present application, the homologues may be proteins or polypeptides having at least about 85% (e.g., having at least about 85%, about 90%, about 91%, about 92%, about 93%, about 94%, about 95%, about 96%, about 97%, about 98%, about 99% or higher) sequence homology with the amino acid sequence of the protein and/or polypeptide (e.g., an antibody or fragment thereof specifically binding to a CD73 protein).

In the present application, the homology typically refers to similarity, likeness or association between two or more sequences. "Percent sequence homology" can be calculated by comparing two sequences to be aligned in a comparison window to determine the number of positions with identical nucleic acid bases (e.g., A, T, C, G, I) or identical amino acid residues (e.g., Ala, Pro, Ser, Thr, Gly, Val, Leu, Ile, Phe, Tyr, Trp, Lys, Arg, His, Asp, Glu, Asn, Gln, Cys, and Met) in the two sequences to obtain the number of matching positions, dividing the number of matching positions by the total number of positions in the comparison window (i.e., window size), and multiplying the result by 100 to generate the percent sequence homology. Alignment for the purpose of determining percent sequence homology can be accomplished in a variety of ways known in the art, for example, using publicly available computer software such as BLAST, BLAST-2, ALIGN or Megalign (DNASTAR) software. One skilled in the art can determine appropriate parameters for aligning sequences, including any algorithms required to achieve maximal alignment within the full-length sequences being compared or within the sequence region of interest. The homology can also be determined by the following methods: FASTA and BLAST. A description of the FASTA algorithm can be found in W. R. Pearson and D. J. Lipman, "Improved Tool for Biological Sequence Comparison", (Proc. Natl. Acad. Sci. ), 85: 2444-2448, 1988; and D. J. Lipman and W. R. Pearson, "Fast and Sensitive Protein Similarity Search", Science, 227: 1435-1441, 1989. A description of the BLAST algorithm can be found in S. Altschul, W. Gish, W. Miller, E. W. Myers and D. Lipman, "Basic Local Alignment Search Tool", Journal of Molecular Biology, 215: 403-410, 1990.

In the present application, the term "include" typically refers to the meaning of include, comprise, contain, or encompass. In some cases, it also means "be" and "consist of".

In the present application, the term "about" typically refers to a change in a range of 0.5-10% greater or less than a specified value, e.g., a change in a range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% greater or less than the specified value.

### DETAILED DESCRIPTION OF THE INVENTION

### IL-36R antibody

In one aspect, the present application provides an antigen binding protein targeting IL-36R. The antigen binding protein targeting IL-36R can maintain a high affinity for IL-36R and has good IL-36R binding activity. The antigen binding protein targeting IL-36R can be used as a surrogate antibody for another IL-36R antibody (the amino acid sequence of HCDR1 is as set forth in SEQ ID NO: 3, the amino acid sequence of HCDR2 is as set forth in SEQ ID NO: 2, the amino acid sequence of HCDR3 is as set forth in SEQ ID NO: 1, the amino acid sequence of VH is as set forth in SEQ ID NO: 4, the amino acid sequence of the heavy chain is as set forth in SEQ ID NO: 11, the amino acid sequence of LCDR1 is as set forth in SEQ ID NO: 7, the amino acid sequence of LCDR2 is as set forth in SEQ ID NO: 6 (STS), the amino acid sequence of LCDR3 is as set forth in SEQ ID NO: 5, the amino acid sequence of VL is as set forth in SEQ ID NO: 8, and the amino acid sequence of the light chain is as set forth in SEQ ID NO: 12) for efficacy studies in mouse models.

In the present application, the antigen binding protein targeting IL-36R may comprise at least one CDR from the heavy chain variable region VH and the light chain variable region VL; the VH may comprise an amino acid sequence as set forth in SEQ ID NO: 30, and the VL may comprise an amino acid sequence as set forth in SEQ ID NO: 34. In the present application, the antigen binding protein targeting IL-36R comprises HCDR1, HCDR2, HCDR3, LCDR1, LCDR2 and LCDR3, the amino acid sequence of the HCDR1 is as set forth in SEQ ID NO: 29, the amino acid sequence of the HCDR2 is as set forth in SEQ ID NO: 28, the amino acid sequence of the HCDR3 is as set forth in SEQ ID NO: 27, the amino acid sequence of the LCDR1 is as set forth in SEQ ID NO: 33, the amino acid sequence of the LCDR2 is as set forth in SEQ ID NO: 32 (AAS), and the amino acid sequence of the LCDR3 is as set forth in SEQ ID NO: 31.

In the present application, the antigen binding protein targeting IL-36R may comprise a heavy chain variable region VH, and the VH may comprise an amino acid sequence as set forth in SEQ ID NO: 30. In the present application, the antigen binding protein targeting IL-36R may comprise a light chain variable region VL, and the VL may comprise an amino acid sequence as set forth in SEQ ID NO: 34. In the present application, the antigen binding protein targeting IL-36R may comprise VH and VL, the VH may comprise an amino acid sequence as set forth in SEQ ID NO: 30, and the VL may comprise an amino acid sequence as set forth in SEQ ID NO: 34.

In the present application, the antigen binding protein targeting IL-36R may comprise an antibody heavy chain constant region. For example, the antibody heavy chain constant region may be derived from an IgG constant region. For example, the antibody heavy chain constant region may comprise an amino acid sequence as set forth in SEQ ID NO: 35.

In the present application, the antigen binding protein targeting IL-3R may comprise an antibody light chain constant region. For example, the antibody light chain constant region may be derived from an Ig kappa constant region. For example, the antibody light chain constant region may comprise an amino acid sequence as set forth in SEQ ID NO: 36.

In the present application, the antigen binding protein targeting IL-36R may comprise an antibody heavy chain, and the antibody heavy chain comprise an amino acid sequence as set forth in SEQ ID NO: 37. In the present application, the antigen binding protein targeting IL-36R may comprise an antibody light chain, and the antibody light chain may comprise an amino acid sequence as set forth in SEQ ID NO: 38. In the present application, the antigen binding protein targeting IL-36R may comprise an antibody heavy chain and an antibody light chain, and the antibody heavy chain may comprise an amino acid sequence as set forth in SEQ ID NO: 37, and the antibody light chain may comprise an amino acid sequence as set forth in SEQ ID NO: 38.

In the present application, the antigen binding protein targeting IL-36R may comprise an antibody or an antigen binding fragment thereof. For example, the antibody comprises a monoclonal antibody, a chimeric antibody, a humanized antibody and/or a fully human antibody. For example, the antigen binding fragment includes Fab, Fab', Fv fragment, F(ab')₂, F(ab)₂, scFv, di-scFv and/or dAb.

### Use, method, indication

The present application provides a method for preventing and/or treating systemic lupus erythematosus by targeting IL-17A and IL-36R dual targets.

In one aspect, the present application provides the use of a drug combination in the preparation of a drug for preventing and/or treating systemic lupus erythematosus, wherein the drug combination comprises an antigen binding protein targeting IL-17A and an antigen binding protein targeting IL-36R.

In another aspect, the present application provides the use of a drug combination in the preparation of a drug for preventing and/or treating systemic lupus erythematosus, wherein the drug combination comprises a nucleic acid molecule encoding an antigen binding protein targeting IL-17A, and a nucleic acid molecule encoding an antigen binding protein targeting IL-36R.

In another aspect, the present application further provides the use of an isolated antigen binding protein in the preparation of a drug, wherein the isolated antigen binding protein comprises a first binding domain targeting IL-36R, and a second binding domain targeting IL-17A, and the drug is used to prevent and/or treat systemic lupus erythematosus. For example, the isolated antigen binding protein is a bispecific antibody, which is capable of targeting IL-36R and IL-17A.

In another aspect, the present application provides a method for preventing and/or treating systemic lupus erythematosus, comprising administering a drug to a subject in need thereof, wherein the drug is capable of targeting IL-17A and IL-36R.

For example, the drug may comprise an antigen binding protein targeting IL-17A and an antigen binding protein targeting IL-36R. For example, the drug may comprise a bi/multi-specific antibody that simultaneously targets IL-17A and IL-36R.

In another aspect, the present application further provides a drug combination for preventing and/or treating systemic lupus erythematosus, wherein the drug combination comprises an antigen binding protein targeting IL-17A and an antigen binding protein targeting IL-36R.

In another aspect, the present application further provides a drug combination for preventing and/or treating systemic lupus erythematosus, the drug combination comprises a nucleic acid molecule encoding an antigen binding protein targeting IL-17A, and a nucleic acid molecule encoding an antigen binding protein targeting IL-36R.

In another aspect, the present application further provides an isolated antigen binding protein for preventing and/or treating systemic lupus erythematosus, the isolated antigen binding protein comprises a first binding domain targeting IL-36R, and a second binding domain targeting IL-17A. For example, the isolated antigen binding protein is a bi/multi-specific antibody targeting IL-17A and IL-36R.

In the present application, the systemic lupus erythematosus may comprise any disease and/or condition associated with systemic lupus erythematosus known in the art. For example, the systemic lupus erythematosus may comprise lupus erythematosus nephritis.

In the present application, the lupus erythematosus nephritis may have one or more of the following characteristics: (1) glomerular lesions; (2) tubulointerstitial lesions; (3) renal vascular lesions; (4) deposits of IgG, IgA, IgM, C3, C4, and/or C1q, along with fibrin, in the kidneys; and (5) deposits in the mesangial area and capillary walls.

In the present application, the survival rate of subjects with systemic lupus erythematosus can be effectively improved by simultaneously targeting IL-17A and IL-36R.

In the present application, the increase in urine protein levels in subjects can be effectively alleviated by simultaneously targeting IL-17A and IL-36R.

In the present application, renal pathological damage caused by systemic lupus erythematosus can be effectively alleviated by simultaneously targeting IL-17A and IL-36R.

In the present application, renal fibrosis caused by systemic lupus erythematosus can be effectively alleviated and/or inhibited by simultaneously targeting IL-17A and IL-36R.

### CDR definition

The CDR of an antibody, also known as the complementary determining region, is a part of the variable region. An amino acid residue in this region may come into contact with an antigen or antigenic epitope. An antibody CDR may be determined by a variety of encoding systems, e.g., CCG, Kabat, Chothia, IMGT, and Kabat/Chothia considered comprehensively. These encoding systems are known in the art, and specific reference may be made to, for example, http://www.bioinf.org.uk/abs/index.html#kabatnum. Those skilled in the art may use different encoding systems to determine the CDR regions according to the sequences and structures of antibodies. Using different coding systems, there may be differences in the CDR region. In the present application, the CDR may include a CDR sequence defined by any CDR definition scheme; it may also include a variant thereof, wherein the variant includes an amino acid sequence of the CDR that has undergone substitution, deletion, and/or addition of one or more amino acids, for example, 1-30, 1-20 or 1-10, and for example, 1, 2, 3, 4, 5, 6, 7, 8 or 9 amino acid substitutions, deletions and/or insertions; it may also include a homolog thereof, wherein the homolog may be an amino acid sequence having at least about 85% (e.g., at least about 85%, about 90%, about 91%, about 92%, about 93%, about 94%, about 95%, about 96%, about 97%, about 98%, about 99% or higher) sequence homology with the amino acid sequence of the CDR.

### Antigen binding protein targeting IL-17A

In the present application, the antigen binding protein targeting IL-17A may comprise at least one CDR from the antibody heavy chain variable region VH, for example, the VH may comprise an amino acid sequence as set forth in SEQ ID NO: 16.

In the present application, the antigen binding protein targeting IL-17A may comprise HCDR3, and the HCDR3 comprises an amino acid sequence as set forth in SEQ ID NO: 13. In the present application, the antigen binding protein targeting IL-17A may comprise HCDR2, and the HCDR2 may comprise an amino acid sequence as set forth in SEQ ID NO: 14. In the present application, the antigen binding protein targeting IL-17A may comprise HCDR1, and the HCDR1 may comprise an amino acid sequence as set forth in SEQ ID NO: 15.

In the present application, the antigen binding protein targeting IL-17A may comprise VH, and the VH comprises an amino acid sequence as set forth in SEQ ID NO: 16.

In the present application, the antigen binding protein targeting IL-17A may comprise at least one CDR from the antibody heavy chain variable region VL, for example, the VL may comprise an amino acid sequence as set forth in SEQ ID NO: 20.

In the present application, the antigen binding protein targeting IL-17A may comprise LCDR3, and the LCDR3 comprises an amino acid sequence as set forth in SEQ ID NO: 17. In the present application, the antigen binding protein targeting IL-17A may comprise LCDR2, and the LCDR2 may comprise an amino acid sequence as set forth in SEQ ID NO: 18 (KVS). In the present application, the antigen binding protein targeting IL-17A may comprise LCDR1, and the LCDR1 may comprise an amino acid sequence as set forth in SEQ ID NO: 19.

In the present application, the antigen binding protein targeting IL-17A may comprise VL, and the VL comprises an amino acid sequence as set forth in SEQ ID NO: 20.

In the present application, the antigen binding protein targeting IL-17A may comprise an antibody or an antigen binding fragment thereof. For example, the antibody comprises a monoclonal antibody, a chimeric antibody, a humanized antibody and/or a fully human antibody. For example, the antigen binding fragment includes Fab, Fab', Fv fragment, F(ab')₂, F(ab)₂, scFv, di-scFv and/or dAb.

In the present application, the antigen binding protein targeting IL-17A may comprise a heavy chain constant region. For example, the heavy chain constant region may be derived from the heavy chain constant region of IgG. For example, the heavy chain constant region may be derived from the heavy chain constant region of human IgG. For example, the heavy chain constant region may be derived from the heavy chain constant region of human IgG1. For example, the heavy chain constant region may be derived from the heavy chain constant region of human IgG4. For example, the heavy chain constant region may comprise an amino acid sequence as set forth in SEQ ID NO: 21.

In the present application, the antigen binding protein targeting IL-17A may comprise a light chain constant region. For example, the light chain constant region may be derived from the light chain constant region of Igκ. For example, the light chain constant region may comprise an amino acid sequence as set forth in SEQ ID NO: 10.

### Antigen binding protein targeting IL-36R

In the present application, the antigen binding protein targeting IL-36R may comprise at least one CDR from the antibody heavy chain variable region VH, for example, the VH may comprise an amino acid sequence as set forth in SEQ ID NO: 4 or SEQ ID NO: 30.

In the present application, the antigen binding protein targeting IL-36R may comprise HCDR3, and the HCDR3 comprises an amino acid sequence as set forth in SEQ ID NO: 1 or SEQ ID NO: 27. In the present application, the antigen binding protein targeting IL-36R may comprise HCDR2, and the HCDR2 may comprise an amino acid sequence as set forth in SEQ ID NO: 2 or SEQ ID NO: 28. In the present application, the antigen binding protein targeting IL-36R may comprise HCDR1, and the HCDR1 may comprise an amino acid sequence as set forth in SEQ ID NO: 3 or SEQ ID NO: 29.

In the present application, the antigen binding protein targeting IL-36R may comprise VH, and the VH comprises an amino acid sequence as set forth in SEQ ID NO: 4 or SEQ ID NO: 30.

In the present application, the antigen binding protein targeting IL-36R may comprise at least one CDR from the antibody heavy chain variable region VL, for example, the VL may comprise an amino acid sequence as set forth in SEQ ID NO: 8 or SEQ ID NO: 34.

In the present application, the antigen binding protein targeting IL-36R may comprise LCDR3, and the LCDR3 comprises an amino acid sequence as set forth in SEQ ID NO: 5 or SEQ ID NO: 31. In the present application, the antigen binding protein targeting IL-36R may comprise LCDR2, and the LCDR2 may comprise an amino acid sequence as set forth in SEQ ID NO: 6 (STS) or SEQ ID NO: 32 (AAS). In the present application, the antigen binding protein targeting IL-36R may comprise LCDR1, and the LCDR1 may comprise an amino acid sequence as set forth in SEQ ID NO: 7 or SEQ ID NO: 33.

In the present application, the antigen binding protein targeting IL-36R may comprise VL, and the VL comprises an amino acid sequence as set forth in SEQ ID NO: 8 or SEQ ID NO: 34.

In the present application, the antigen binding protein targeting IL-36R may comprise an antibody or an antigen binding fragment thereof. For example, the antibody comprises a monoclonal antibody, a chimeric antibody, a humanized antibody and/or a fully human antibody. For example, the antigen binding fragment includes Fab, Fab', Fv fragment, F(ab')₂, F(ab)₂, scFv, di-scFv and/or dAb.

In the present application, the antigen binding protein targeting IL-36R may comprise a heavy chain constant region. For example, the heavy chain constant region may be derived from the heavy chain constant region of IgG. For example, the heavy chain constant region may be derived from the heavy chain constant region of human IgG. For example, the heavy chain constant region may be derived from the heavy chain constant region of human IgG1. For example, the heavy chain constant region may be derived from the heavy chain constant region of human IgG4. For example, the heavy chain constant region may comprise an amino acid sequence as set forth in SEQ ID NO: 9 or SEQ ID NO: 35.

In the present application, the antigen binding protein targeting IL-36R may comprise a light chain constant region. For example, the light chain constant region may be derived from the light chain constant region of Igκ. For example, the light chain constant region may comprise an amino acid sequence as set forth in SEQ ID NO: 10 or SEQ ID NO: 36.

### Bi/multispecific antibodies

In the present application, the multispecific antibody is capable of simultaneously targeting IL-17A and IL-36R. For example, the multispecific antibody may be a bispecific antibody. For example, the IL-17A can be human IL-17A. For example, the IL-36R can be human IL-36R.

In the present application, the multispecific antibody may comprise a targeting moiety (first binding domain) for IL-17A and a targeting moiety (second binding domain) for IL-36R. In the present application, the multispecific antibody may have one or more of the following properties: (1) binding to IL-36R with high affinity; (2) binding to IL-1Rrp2 with high affinity; (3) blocking the binding of IL-36α, IL-36β, and IL-36γ to IL-36R; and (4) blocking IL-36α-, IL-36β-, and IL-36γ-mediated cytokine secretion.

In the present application, the targeting moiety for IL-17A may comprise at least one CDR from the antibody heavy chain variable region VH. For example, the VH may comprise an amino acid sequence as set forth in SEQ ID NO: 16.

In the present application, the targeting moiety for IL-17A may comprise HCDR3, and the HCDR3 comprises an amino acid sequence as set forth in SEQ ID NO: 13.

In the present application, the targeting moiety for IL-17A may comprise HCDR2, and the HCDR2 comprises an amino acid sequence as set forth in SEQ ID NO: 14.

In the present application, the targeting moiety for IL-17A may comprise HCDR1, and the HCDR1 comprises an amino acid sequence as set forth in SEQ ID NO: 15.

In the present application, the targeting moiety for IL-17A may comprise HCDR1, HCDR2 and HCDR3, and the HCDR1 comprises an amino acid sequence as set forth in SEQ ID NO: 15, the HCDR2 comprises an amino acid sequence as set forth in SEQ ID NO: 14, and the HCDR3 comprises an amino acid sequence as set forth in SEQ ID NO: 13.

In the present application, the targeting moiety for IL-17A may comprise VH, and the VH may comprise an amino acid sequence as set forth in SEQ ID NO: 16.

For example, the targeting moiety for IL-17A may comprise at least one CDR from the antibody light chain variable region VL. For example, the VL may comprise an amino acid sequence as set forth in SEQ ID NO: 20.

In the present application, the targeting moiety for IL-17A may comprise LCDR3, and the LCDR3 comprises an amino acid sequence as set forth in SEQ ID NO: 17.

In the present application, the targeting moiety for IL-17A may comprise LCDR2, and the LCDR2 comprises an amino acid sequence as set forth in SEQ ID NO: 18 (KVS).

In the present application, the targeting moiety for IL-17A may comprise LCDR1, and the LCDR1 comprises an amino acid sequence as set forth in SEQ ID NO: 19.

In the present application, the targeting moiety for IL-17A may comprise LCDR1, LCDR2 and LCDR3, and the LCDR1 comprises an amino acid sequence as set forth in SEQ ID NO: 19, the LCDR2 comprises an amino acid sequence as set forth in SEQ ID NO: 18 (KVS), and the LCDR3 comprises an amino acid sequence as set forth in SEQ ID NO: 17.

In the present application, the targeting moiety for IL-17A may comprise VL, and the VL may comprise an amino acid sequence as set forth in SEQ ID NO: 20.

In the present application, the targeting moiety for IL-17A may be an IL-17A antibody. For example, the targeting moiety for IL-17A may further comprise an antibody heavy chain constant region. For example, the targeting moiety for IL-17A may further comprise an antibody light chain constant region.

In the present application, the heavy chain constant region of the IL-17A antibody may comprise an amino acid sequence as set forth in SEQ ID NO: 21. In the present application, the light chain constant region of the IL-17A antibody may comprise an amino acid sequence as set forth in SEQ ID NO: 10.

For example, the targeting moiety for IL-36R may comprise at least one CDR from the antibody heavy chain variable region VH. For example, the VH may comprise an amino acid sequence as set forth in SEQ ID NO: 4 or SEQ ID NO: 30.

In the present application, the targeting moiety for IL-36R may comprise HCDR3, and the HCDR3 comprises an amino acid sequence as set forth in SEQ ID NO: 1 or SEQ ID NO: 27.

In the present application, the targeting moiety for IL-36R may comprise HCDR2, and the HCDR2 comprises an amino acid sequence as set forth in SEQ ID NO: 2 or SEQ ID NO: 28.

In the present application, the targeting moiety for IL-36R may comprise HCDR1, and the HCDR1 comprises an amino acid sequence as set forth in SEQ ID NO: 3 or SEQ ID NO: 29.

In the present application, the targeting moiety for IL-36R may comprise HCDR1, HCDR2 and HCDR3, and the HCDR1 comprises an amino acid sequence as set forth in SEQ ID NO: 3 or SEQ ID NO: 29, the HCDR2 comprises an amino acid sequence as set forth in SEQ ID NO: 2 or SEQ ID NO: 28, and the HCDR3 comprises an amino acid sequence as set forth in SEQ ID NO: 1 or SEQ ID NO: 27.

In the present application, the targeting moiety for IL-36R may comprise VH, and the VH may comprise an amino acid sequence as set forth in SEQ ID NO: 4 or SEQ ID NO: 30.

For example, the targeting moiety for IL-36R may comprise at least one CDR from the antibody light chain variable region VL. For example, the VL may comprise an amino acid sequence as set forth in SEQ ID NO: 8 or SEQ ID NO: 34.

In the present application, the targeting moiety for IL-36R may comprise LCDR3, and the LCDR3 comprises an amino acid sequence as set forth in SEQ ID NO: 5 or SEQ ID NO: 31.

In the present application, the targeting moiety for IL-36R may comprise LCDR2, and the LCDR2 comprises an amino acid sequence as set forth in SEQ ID NO: 6 (STS) or SEQ ID NO: 32 (AAS).

In the present application, the targeting moiety for IL-36R may comprise LCDR1, and the LCDR1 comprises an amino acid sequence as set forth in SEQ ID NO: 7 or SEQ ID NO: 33.

In the present application, the targeting moiety for IL-36R may comprise LCDR1, LCDR2 and LCDR3, and the LCDR1 comprises an amino acid sequence as set forth in SEQ ID NO: 7 or SEQ ID NO: 33, the LCDR2 comprises an amino acid sequence as set forth in SEQ ID NO: 6 (STS) or SEQ ID NO: 32 (AAS), and the LCDR3 comprises an amino acid sequence as set forth in SEQ ID NO: 5 or SEQ ID NO: 31.

In the present application, the targeting moiety for IL-36R may comprise VL, and the VL may comprise an amino acid sequence as set forth in SEQ ID NO: 8 or SEQ ID NO: 34.

In the present application, the targeting moiety for IL-36R may be scFv. For example, the VH and VL may be linked via a linking peptide. For example, the scFv may comprise an amino acid sequence as set forth in SEQ ID NO: 24.

In a specific embodiment, the bispecific antibody is formed by fusion of the anti-IL-17A antibody and the ScFv of the anti-IL-36R antibody, and has two pairs of peptide chains that are symmetrical to each other; each pair of peptide chains comprises a light chain L chain and a heavy chain H chain; all the peptide chains are linked via disulfide bonds, and any pair of peptide chains has a structure of the H chain and the L chain represented by formula I from N-terminus to C-terminus, 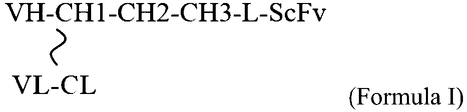 wherein,
VH represents the heavy chain variable region of the anti-IL-17A antibody;
VL represents the light chain variable region of the anti-IL-17A antibody;
CH1, CH2 and CH3 represent the heavy chain constant regions CH1, CH2 and CH3 of the anti-IL-17A antibody, respectively;
CL represents the light chain constant region of the anti-IL-17A antibody;
ScFv represents the ScFv of the anti-IL-36R antibody;
L is a linker element;
"~" represents a disulfide bond;
"-" represents a peptide bond;
wherein, the bispecific antibody has an activity of binding to IL-17A and IL-36R simultaneously.

For example, The H chain of the bispecific antibody has an amino acid sequence as set forth in SEQ ID NO: 25; and the L chain of the bispecific antibody has an amino acid sequence as set forth in SEQ ID NO: 23.

The bispecific antibody of the present application includes not only intact antibodies, but also fragments of antibodies having immunological activity or fusion proteins formed by antibodies and other sequences. Therefore, the present application further includes fragments, derivatives and analogs of the antibodies.

The bispecific antibody of the present application refers to an antibody having anti-IL-17A and anti-IL-36R activity, and comprising two structures of the aforementioned Formula I. The term further includes variant forms of antibodies having the same function as the bispecific antibody of the present application and comprising two structures of the aforementioned Formula I. These variant forms include (but are not limited to): one or more (usually 1-50, for example, 1-30, for example, 1-20, for example, 1-10) amino acid deletions, insertions and/or substitutions, and addition of one or more (usually within 20, for example, within 10, for example, within 5) amino acids at the C-terminus and/or N-terminus. For example, in the art, substitution with amino acids having close or similar properties usually does not change the function of the protein. For another example, addition of one or more amino acids at the C-terminus and/or N-terminus typically does not change the function of the protein. The term also includes active fragments and active derivatives of the bispecific antibodies of the present application.

### Drugs

In the present application, the drug may comprise an active molecule and, optionally, a pharmaceutically acceptable carrier. Typically , these substances may be formulated in a non-toxic, inert and pharmaceutically acceptable aqueous carrier medium, wherein the pH is typically about 5-8, for example, the pH may be about 6-8, although the pH value may vary depending on the nature of the formulated substance and the condition to be treated. The formulated drug may be administered by conventional routes, including (but not limited to): intraperitoneal, intravenous, or topical administration.

The drug of the present application comprises a safe and effective amount (such as 0.001-99 wt%, such as 0.01-90 wt%, such as 0.1-80 wt%) of the active ingredient described above in the present application and a pharmaceutically acceptable carrier or excipient. Such carriers include (but are not limited to): saline, buffer, glucose, water, glycerol, ethanol, and combinations thereof. The drug formulation should match the method of administration. The pharmaceutical composition of the present application may be prepared in the form of an injection, for example, by conventional methods using physiological saline or an aqueous solution containing glucose and other adjuvants. Pharmaceutical compositions such as injections and solutions are preferably prepared under sterile conditions. The dosage of the active ingredient is a therapeutically effective amount, for example, about 1 µg/kg body weight to about 5 mg/kg body weight per day. In addition, the polypeptide of the present application may also be used with other therapeutic agents.

When using a pharmaceutical composition, a safe and effective amount of the pharmaceutical composition is administered to a mammal, wherein the safe and effective amount is usually at least about 10 µg/kg body weight, and in most cases does not exceed about 50 mg/kg body weight, for example, the dose may be about 10 µg/kg body weight to about 20 mg/kg body weight. Of course, the specific dosage should also take into account factors such as the route of administration and the patient's health status, which are all within the skill range of skilled physicians.

Without intending to be bound by any theory, the following examples are only intended to illustrate the antigen binding protein, preparation method and use of the present application, etc., and are not intended to limit the scope of the invention of the present application.

### EXAMPLES

### Example 1 Development of surrogate antibodies for the IL-36R antibody of the present application for mouse models

The anti-human IL-36R antibody provided in the present application (HB0034: the amino acid sequence of HCDR1 is as set forth in SEQ ID NO: 3, the amino acid sequence of HCDR2 is as set forth in SEQ ID NO: 2, the amino acid sequence of HCDR3 is as set forth in SEQ ID NO: 1, the amino acid sequence of VH is as set forth in SEQ ID NO: 4, the amino acid sequence of the heavy chain is as set forth in SEQ ID NO: 11, the amino acid sequence of LCDR1 is as set forth in SEQ ID NO: 7, the amino acid sequence of LCDR2 is as set forth in SEQ ID NO: 6 (STS), the amino acid sequence of LCDR3 is as set forth in SEQ ID NO: 5, the amino acid sequence of VL is as set forth in SEQ ID NO: 8, and the amino acid sequence of the light chain is as set forth in SEQ ID NO: 12) does not bind to mice, so a surrogate antibody for human IL-36R is developed for drug efficacy testing experiments in mouse models.

### 1.1 Preparation of hybridoma cells producing mouse monoclonal antibodies

The method for preparing mouse-derived monoclonal antibodies adopts the hybridoma preparation technology invented by Kohler and Milstein in 1975 (Nature, 1975, 256: 495-497). First, the IL-1Rrp2 gene of C57BL/6 mice was knocked out, and 5 mice that were confirmed not to express the gene were selected for immunization. Mouse IL-1Rrp2 protein (R&D, #2354-RP/CF) was emulsified with an immune adjuvant, and 5 IL-1Rrp2-knockout mice were immunized respectively. After four rounds of immunization, serum was collected to detect the titer using ELISA, and spleen cells from five mice were fused with SP2/0 myeloma cells respectively. After HAT screening of hybridoma cells, the cell culture supernatant was tested for mouse IL-36R binding activity, high-affinity antibodies were selected through Biacore detection, and the antibodies were screened for their inhibitory effects on IL-6 secretion by mouse IL-36 β/γ stimulated BMDC. The best hybridoma monoclonal cell lines including 44G8 were finally selected for sequence analysis (as shown in Table 1).

**Table 1 Sequence analysis results of hybridoma monoclonal cell lines**

| **NO** | **Clone** | **ELISA Binding moIL36R** | **Mo-IL36R affinity determination** | | | **BMDC-IL-36β inhibition rate** | **BMDC-IL-36γ inhibition rate** |
|---|---|---|---|---|---|---|---|
| | | | **ka (1/Ms)** | **kd (1/s)** | **Mo-IL36R KD (M)** | | |
| 1 | 102F7 | 3.44 | 2.13E+06 | 6.34E-03 | 2.98E-09 | 57.80% | 21.56% |
| 2 | 76A1 | 3.22 | 1.30E+04 | 2.88E-06 | 2.22E-10 | 74.42% | 89.03% |
| 3 | 33B6 | 2.85 | 7.62E+05 | 1.41E-02 | 1.84E-08 | 64.05% | 37.68% |
| 4 | 77E10 | 3.65 | 2.41E+05 | 3.47E-04 | 1.44E-09 | 44.24% | 29.02% |
| 5 | 71E3 | 3.18 | 4.63E+04 | 7.66E-04 | 1.65E-08 | 80.48% | 94.47% |
| 6 | 44G8 | 3.01 | 9.01E+04 | 4.54E-04 | 5.04E-09 | 77.22% | 96.23% |

### 1.2 Extraction of the gene of the antibody variable region of the best hybridoma monoclonal cell line 44G8

First, total RNA was extracted from antibody-positive hybridoma cells using a total RNA extraction kit (Qiagen, 74181), and cDNA was obtained by reverse transcription using a 5' cDNA end rapid amplification (5' RACE) kit (Takara, 634859). Then, primers specific to the variable region gene of the antibody were designed, and the variable region gene of the antibody was amplified by two rounds of PCR using the cDNA as a template. The gene of interest was cloned into the vectors 000005 (light chain mouse Igκ) and 000007 (heavy chain mouse IgG 2a, with Fc having D265A N297A (EU numbering, referred to as DANA) mutations to weaken the binding of the antibody to FcγR, eliminate ADCC/CDC effects, and remove C-terminal lysine K) containing a mouse antibody constant region gene by homologous recombination using In-Fusion Snap Assembly Master Mix (Takara, 638949). Then, the constructed plasmid was transformed into Escherichia coli Stellar^{™}Competent Cells competent strain (Takara, 636736), single clones were selected and sent to Suzhou Azenta Biotechnology Co., Ltd. (referred to as Azenta) for Sanger sequencing; finally, the amino acid sequence of the antibody variable region was obtained by translation based on the sequencing DNA sequence results.
>VH (SEQ ID NO: 39) 
EVQLQQSGPELVKPGASVKMSCKASGYTFTDYNMHWVKQSHGKSLEWIGFFNPNN
GGTRYNQKFKGKATLTVNKSSSTAYMDLRSLTSEDSAVYYCTRQEIYYSNYFDYWGQG
TTLTVSS
>VL (SEQ ID NO: 34)

DIVLTQSPASLAVSLGQRATISCKASQSVDYDGDSYMNWYQQKPGQPPKLLIYAASN
LESGIPARFSGSGSGTDFTLNIHPVEEEDAATYSCQQSYEDPYTFGGGAKLEIK

### 1.3 Preparation of HB0034SA antibody

After the monoclonal sequencing plasmids of the heavy and light chains of 44G8 with correct sequencing were returned from Azenta, PCR primers were designed using the returned plasmids as templates to mutate the potential glycosylation site at position 73 of the heavy chain variable region, i.e., asparagine N, to glutamine Q. The full-length sequence of the mutated antibody gene (see below for the sequence) was amplified and cloned into the mammalian cell expression vector pUKWO. The obtained light chain and heavy chain vectors were numbered as 300700 and 300701, respectively. Plasmids were extracted using an endotoxin-free plasmid extraction kit (TIANGEN Biotech (Beijing) Co., Ltd., DP117). The heavy chain and light chain plasmids were mixed with polyetherimide PEI (Polysciences, 24765-1) at a mass concentration ratio of 1:2 to form liposome complexes, which were then transfected into CHO-S cells (Cat. No.: R80007, Thermo Fisher Scientific (China) Co., Ltd.). After low-temperature induction expression culture at 32°C for 7 days, the cell culture supernatant was collected and purified by Protein A affinity chromatography to obtain the chimeric antibody HB0034SA. For HB0034SA, the amino acid sequence of HCDR1 was as set forth in SEQ ID NO: 29, the amino acid sequence of HCDR2 was as set forth in SEQ ID NO: 28, the amino acid sequence of HCDR3 was as set forth in SEQ ID NO: 27, the amino acid sequence of VH was as set forth in SEQ ID NO: 30, the amino acid sequence of the heavy chain was as set forth in SEQ ID NO: 37, the amino acid sequence of LCDR1 was as set forth in SEQ ID NO: 33, the amino acid sequence of LCDR2 was as set forth in SEQ ID NO: 32 (AAS), the amino acid sequence of LCDR3 was as set forth in SEQ ID NO: 31, the amino acid sequence of VL was as set forth in SEQ ID NO: 34, and the amino acid sequence of the light chain was as set forth in SEQ ID NO: 38.

### Example 2 Study on HB0034SA for substitutability

The HB0034 antibody can specifically bind to the human IL-36R protein, but does not bind to the IL-36R protein of rabbit, rat, and mouse. HB0034 is a monoclonal antibody that can specifically bind to human IL-36R obtained by panning mice immunized with human IL-36R. The HB0034 antibody is selected for clinical administration.

HB0034SA is an anti-mouse IL-36R monoclonal antibody independently developed by the applicant and obtained by panning rats immunized with Mouse IL-36R. It is intended to be used as a surrogate antibody for HB0034.

### 2.1 Comparison of the affinity of HB0034SA and HB0034 for mouse IL-36R (also known as IL-1 Rrp2) and human IL-36R, respectively

The affinity determination results showed that the affinity KD value of HB0034SA for mouse IL-36R was 5.68 × 10⁻¹⁰ M, and the affinity KD value of HB0034 for human IL-36R was 3.25 × 10⁻¹⁰ M. The affinity of HB0034 for human IL-36R was comparable to the affinity of HB0034SA for mouse IL-36R.

### 2.2 Comparison of binding activity of HB0034SA and HB0034 with mouse IL-36R (also known as IL-1 Rrp2) and human IL-36R, respectively

ELISA results showed that under the condition of 1 µg/mL antigen coating, the binding EC₅₀ of HB0034SA to mouse IL-36R was 25.380 ng/mL, both HB0034 and HB0034SA had a molecular weight around 150 kDa, and the binding EC₅₀ of HB0034 to human IL-36R was 12.120 ng/mL (a two-fold difference in EC₅₀ values), so it could be considered that the binding capability of HB0034SA to the corresponding target protein was comparable to that of HB0034.

### 2.3 Comparative study of in vitro biological activities of HB0034SA and HB0034

The biological activities of HB0034 and HB0034SA in inhibiting the secretion of cytokines (IL-6) by primary cells were measured.

The experimental results showed that HB0034 could inhibit the binding of Recombinant Human IL-36α to human IL-36R, and reduce the amount of IL-36α-mediated IL-6 secretion by human monocyte-derived macrophage (MDM). The IC₅₀ of the inhibitory effect of the antibody was 0.0413 nM. Similarly, HB0034SA could inhibit the binding of Recombinant Mouse IL-36α to mouse IL-36R, and reduce the amount of IL-36α-mediated IL-6 secretion by mouse bone marrow-derived dendritic cells (BMDC). The IC₅₀ of the inhibitory effect of the antibody was 0.0806 nM. In summary, under the experimental conditions, HB0034 had the biological activity of inhibiting human IL-36α and human IL36R receptor mediation, and HB0034SA had the biological activity of inhibiting mouse IL-36α and mouse IL36R receptor mediation, and the IC₅₀ of the two were not much different (a 2-fold difference in IC50), indicating that the biological functional activities of the two were comparable.

In summary, the target affinity results showed that the affinity of HB0034 for human IL-36R was slightly higher than that of HB0034SA for mouse IL-36R (about 47 times higher), but the binding activity results showed that the binding capability of HB0034SA to the corresponding target protein was comparable to that of HB0034. The biological functional activity results showed that HB0034/HB0034SA inhibited the secretion of IL-6 in cells mediated by the binding of human/mouse IL-36α to human/mouse IL-36R, with IC₅₀ values of 0.0413 nM and 0.0806 nM, respectively. The biological functional activities of the two were comparable. Since the functional activity of the Fc terminal was not a key quality attribute of HB0034, we mainly focused on the comparison results of biological activity, binding activity and affinity for the target protein in evaluating whether HB0034SA could be used as a surrogate in non-clinical pharmacodynamic studies. Combining the comparison results of biological activity, binding activity and affinity for the target protein, it could be considered that HB0034SA could be used as a surrogate.

### Example 3 Functional inhibition by combined stimulation of IL-17A antibody and IL-36R antibody

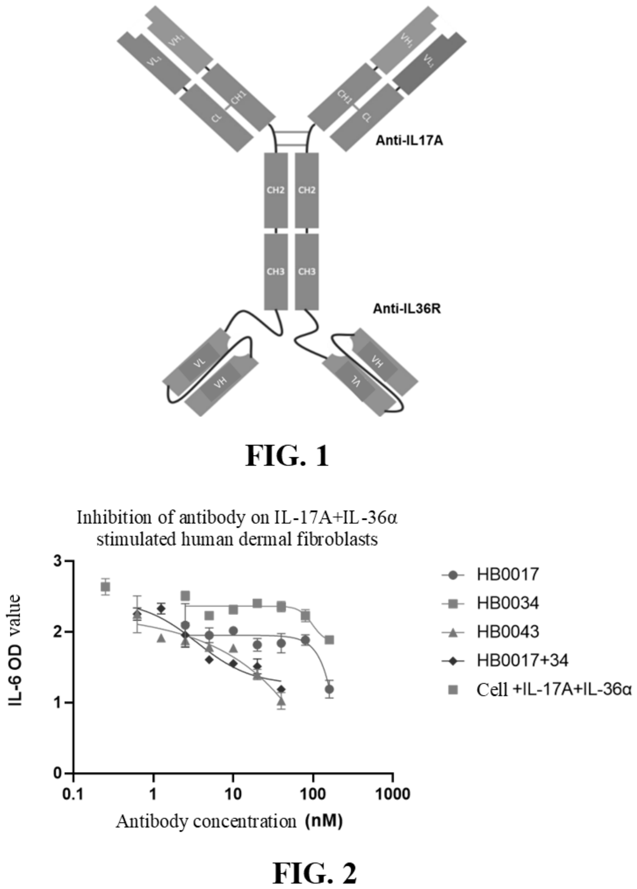

HB0043 is a bispecific antibody targeting IL-17A and IL-36R, wherein the IL-17A targeting moiety of HB0043 uses the VH and VL of HB0017, and the IL-36R targeting moiety of HB0043 uses the VH and VL of HB0034. The structural pattern of HB0043 belongs to the Morrison pattern (IgG-scFv), that is, both the C-termini of the two heavy chains of an IgG antibody are linked to the scFv fragment of another antibody, and the structural diagram is shown in FIG. 1. The amino acid sequence of the heavy chain of HB0043 is as set forth in SEQ ID NO: 25, and the amino acid sequence of the light chain is as set forth in SEQ ID NO: 23.

Normal human dermal fibroblasts (NHDF) were digested, resuspended in culture medium (DMEM+ 10% FBS + 1% P/S) at a concentration of 3 × 10⁵/mL, seeded in a 96-well plate at 100 µL/well, and incubated overnight at 37°C and 5% CO₂. After the culture medium was replaced with basal culture medium for 16 hours of cell starvation treatment, IL-17A was diluted from 1000 ng/ml to nine concentrations in a five-fold concentration gradient using normal culture medium, and IL-36α, β and γ were diluted from 1000 ng/ml to nine concentrations in a three-fold concentration gradient and stimulated NHDF for 24 hours for induction. The cell supernatant was obtained by centrifugation and the protein concentrations of IL-6 and IL-8 in the supernatant were detected by Elisa, and the EC₅₀ of IL-17A and IL-36 ligands were calculated. NHDF cells were plated in a 96-well plate at 3 × 10⁴ per well and cultured overnight in an incubator. The cells were stimulated with 15 ng/ml IL-36α, 1 ng/ml IL-36β or 2 ng/ml IL-36γ + 0.5 ng/ml IL-17A, and 2-fold serial dilutions of HB0034, HB0017, HB0043 and HB0017 + HB0034 antibodies were mixed with cytokines in a 200 µL system, with initial concentrations of 160, 160, 40 and 40+40 nM, respectively, and incubated overnight at 37°C and 5% CO₂. The supernatant was collected after 6-fold dilution, and the concentration of IL-6/8 cytokines was detected using a human IL-6/8 ELISA kit (Biolegend, Cat# 430515/431501), and the antibody concentration-response curve was determined.

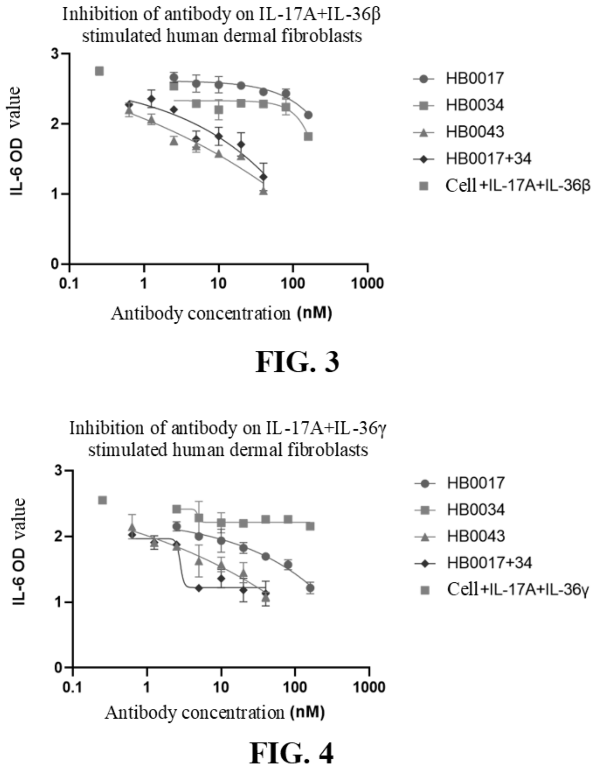

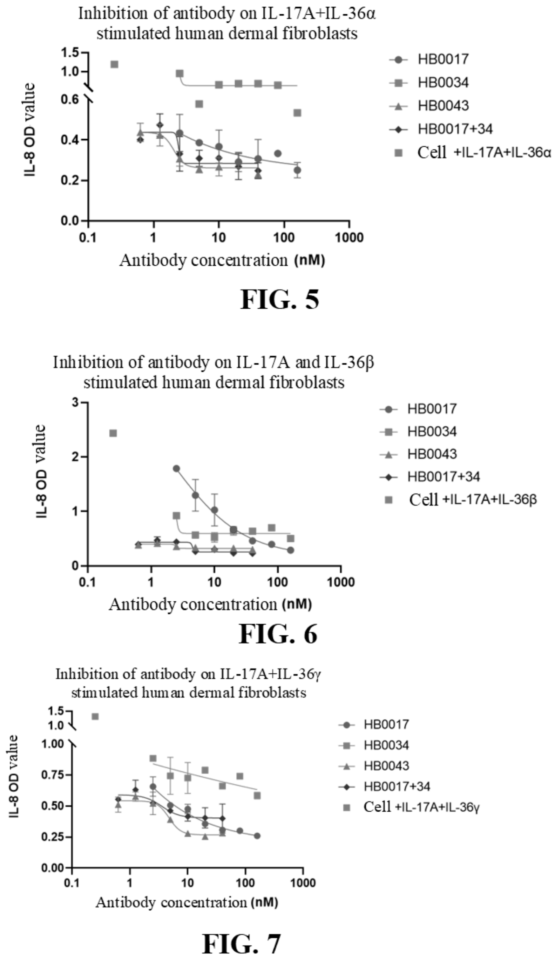

The results were shown in FIGs. 2-7. The results showed that HB0043 or HB0017 combined with HB0034 could effectively block the joint stimulation of IL-36 ligand and IL-17A for NHDF to secrete IL-6 and IL-8. According to the antibody concentration-response curve, HB0043 significantly inhibited the secretion of IL-6 and IL-8 induced by these cytokines in a dose-dependent manner. Compared with HB0017 and HB0034, HB0043 had a better effect in inhibiting the secretion of IL-6 and IL-8 at the same concentration, and achieved similar effects at lower concentrations. The combination of HB0017 and HB0034 showed similar effects to HB0043. In summary, our data showed that the HB0043 bispecific antibody and the combination of two monoclonal antibodies (HB0017 and HB0034) could more effectively block the secretion of IL-6 and IL-8 induced by IL-17A and IL-36 ligands than single monoclonal antibodies, thereby potentially playing a better role in dual inhibition on IL-17A and IL-36-mediated pathways in vitro.

### Example 4 Evaluation of the efficacy of IL-17A antibody and IL-36R antibody and their combination in inhibiting UUO-induced renal fibrosis in mice

36 12-week-old C57BL/6 male mice were randomly divided into 6 groups according to their initial body weight, with 6 mice in each group. They underwent UUO surgery. The isotype control group was administered 900543 (IgG1 (Fc silencing) negative control antibody, which targeted small molecule TNP, 30 mg/kg, i.p.), the positive drug group was administered Paricalcitol (0.1 µg/kg, i.p.), the combination group was administered HB0017 + HB0034SA (15 mg/kg, i.p.+ 15 mg/kg, i.p.), the single drug group was administered HB0017 (30 mg/kg, i.p.), and another single drug group was administered HB0034SA (30 mg/kg, i.p.), normal control group had no surgical treatment. Before UUO surgery, the drug was administered once. On the day of surgery, the left ureter of the mouse was ligated using inhalation anesthesia. After surgery, the drug was administered every other day, 3 times a week, for 2 weeks. Before sampling, the animal body weight was measured. Blood was collected, serum was aliquoted and stored at -80°C. The kidney on the operative side was removed, weighed, and preserved in formalin (for tissue staining).

Commercial kits were used to detect serum creatinine (Cr) and serum urea nitrogen (BUN) levels. And renal tissue staining and immunohistochemical staining were performed.

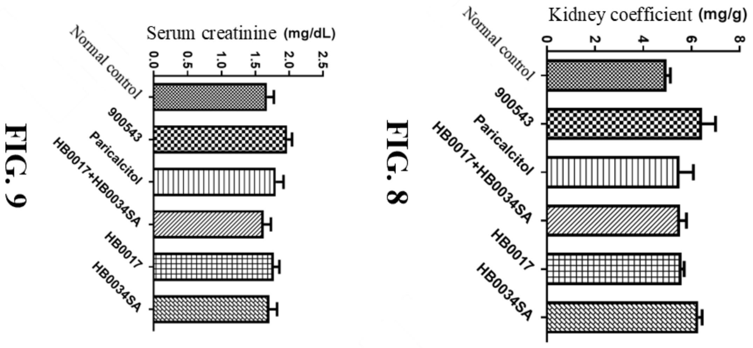

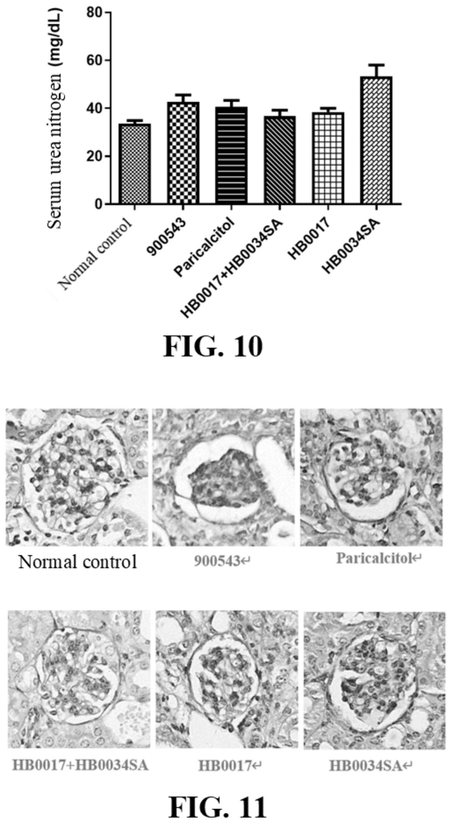

The results were shown in FIG. 8. Compared with the normal control group, the kidney coefficient of the model group increased by 29.8%; compared with the 900543 model group, the kidney coefficient of the positive drug Pari group decreased by 14.4%, and HB0017 + HB0034SA (14.3%) showed a further downward trend compared with the single drug groups (HB0017: 13.2%; HB0034SA: 2.5%). FIG. 9 shows that there was no statistical difference in blood Cr between the groups, compared with the 900543 model group (increased by 17.9%, vs. normal control), the combined drug group HB0017 + HB0034SA (17.8%) showed a further downward trend compared with the single drug groups (HB0017: 10.1%; 900971: 13.7%). Similarly, FIG. 10 shows that there was no statistical difference in blood BUN between the groups. Among the drug-treated groups, the lowest value of blood BUN appeared in the HB0017 + HB0034SA combination group, and blood BUN decreased by 14.1% compared with the model group.

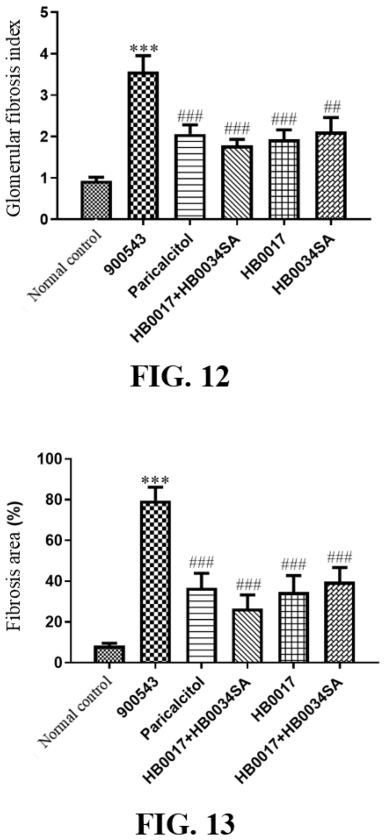

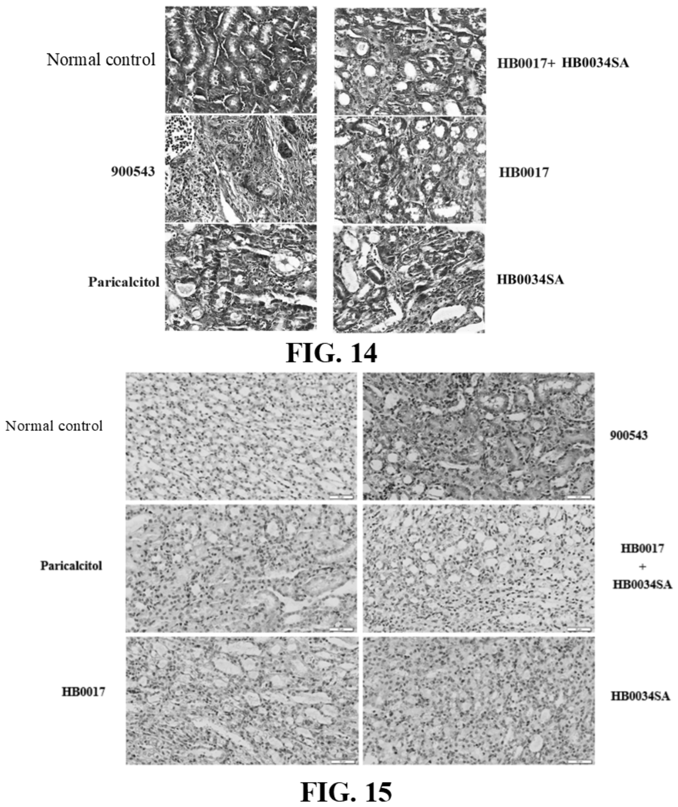

Next, the improvement effect of the test antibody on renal fibrosis was evaluated by PAS staining and Masson staining. PAS staining and quantitative scoring mainly reflected glomerular fibrosis. As shown in FIG. 11, the dark blue-purple color in the glomerulus indicates positive fibrosis. The quantitative analysis results in FIG. 12 show that UUO successfully induced significant glomerular fibrosis (P < 0.001). Compared with the model group, the positive drug (P < 0.001) and the test antibody could significantly inhibit glomerular fibrosis, and the combined drug group HB0017 + HB0034SA exhibited better effect than their respective single drug HB0017 or HB0034SA. Masson staining and quantification of fibrosis area could reflect the progression of tubulointerstitial fibrosis. As shown in FIG. 13, the blue positive signal in the tubulointerstitium indicates fibrotic matrix. The quantitative analysis results in FIG. 14 showed that UUO significantly caused tubulointerstitial fibrosis in mice (P < 0.001). Compared with the model group, the positive drug and different test antibody intervention schemes could significantly inhibit tubulointerstitial fibrosis (P < 0.01). Similarly, the combined drug group HB0017 + HB0034SA exhibited the best effect.

In addition, to further clarify the anti-renal fibrosis effect of the test antibody, immunohistochemistry was used to detect the positive expression of renal fibronectin (FN). FN is an important component of the extracellular matrix and fibrosis matrix protein, and is often used to characterize the phenotype of tissue fibrosis. As shown in FIG. 15, UUO induced a large amount of expression and aggregation of tubulointerstitial FN, which once again showed that the kidneys of UUO mice had obvious fibrosis. Each drug group, including Pari and the test antibody, could reduce the positive expression signal of FN, indicating that the drug had the effect of inhibiting and downregulating FN expression, and had a good biological effect of preventing and treating UUO-induced renal fibrosis.

### Example 5 Therapeutic effect of the combination of IL-17A antibody and IL-36R surrogate antibody on lupus nephritis in spontaneous lupus erythematosus mice (NZB/NZW F1)

50 NZB/NZW F1 mice were randomly divided into 5 groups according to the animal ANA level, anti-dsDNA level, urine protein and initial body weight, with 10 mice in each group, namely HB0017 single drug group (human IL-17A monoclonal antibody, 50 mg/kg, i.p.), HB0034SA single drug group (mouse IL-36R monoclonal antibody, 50 mg/kg, i.p.), HB0017 + HB0034SA combination group (25 + 25 mg/kg, i.p.), positive drug group (prednisone, 9 mg/kg, p.o.) and isotype control group (IgG1 50 mg/kg, i.p.). Another 10 C57BL/6 mice served as the negative control group. Among them, for HB0017 antibody, the amino acid sequence of HCDR1 was as set forth in SEQ ID NO: 15, the amino acid sequence of HCDR2 was as set forth in SEQ ID NO: 14, the amino acid sequence of HCDR3 was as set forth in SEQ ID NO: 13, the amino acid sequence of LCDR1 was as set forth in SEQ ID NO: 19, the amino acid sequence of LCDR2 was as set forth in SEQ ID NO: 18, the amino acid sequence of LCDR3 was as set forth in SEQ ID NO: 17, the amino acid sequence of VH was as set forth in SEQ ID NO: 16, the amino acid sequence of VL was as set forth in SEQ ID NO: 20, the amino acid sequence of heavy chain was as set forth in SEQ ID NO: 22, and the amino acid sequence of antibody light chain was as set forth in SEQ ID NO: 23. HB0034SA antibody was the antibody described in example 1.

When NZB/NZW F1 mice were 30 weeks old, the levels of ANA and anti-dsDNA were significantly increased. The animals in the isotype group, HB0017, HB0034SA and HB0017 + HB0034SA combination groups were administered by intraperitoneal injection, and the model group was administered with normal saline by intraperitoneal injection. The frequency of administration was twice a week, and the administration lasted for 10 to 40 weeks. The positive control group was administered by gavage once a day.

The body weight was weighed twice a week, and random urine was collected once a week to detect urine protein concentration. At the end of the experiment, the left kidney of the animal was collected, fixed and sliced for pathological scoring.

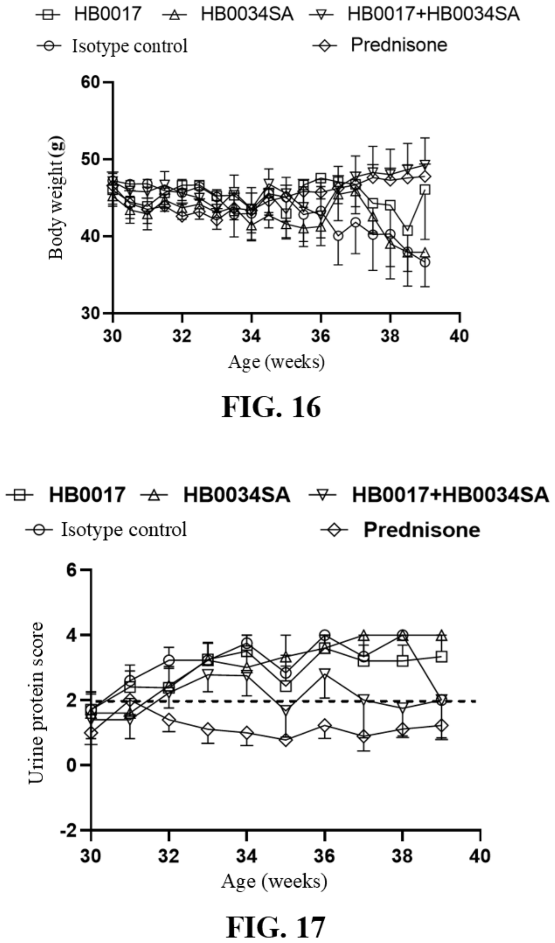

The weight change results of NZB/NZW F1 mice are shown in FIG. 16. The results showed that the combined intraperitoneal injection of HB0017 + HB0034SA could effectively improve the weight loss of mice.

The results of urine protein level detection of NZB/NZW F1 mice are shown in FIG. 17. The results showed that administration by intraperitoneal injection in HB0017 + HB0034SA group could delay and inhibit the increase of urine protein level in NZB/NZW F1 mice.

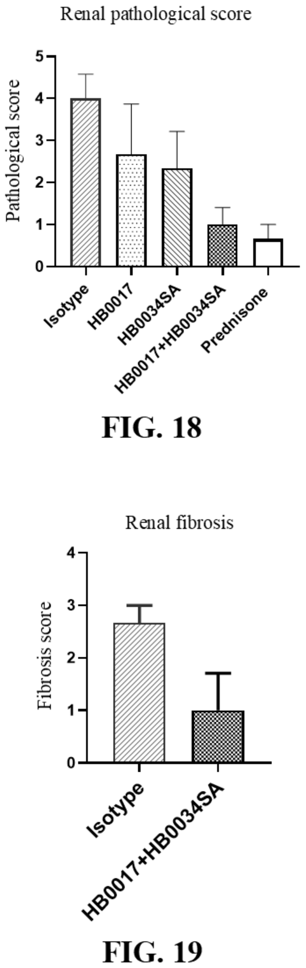

The results of the renal pathological damage evaluation of NZB/NZW F1 mice are shown in FIG. 18. The results showed that administration by intraperitoneal injection in HB0017 + HB0034SA group could significantly improve the renal pathological damage in NZB/NZW F1 mice.

The results of the renal fibrosis evaluation of NZB/NZW F1 mice are shown in FIG. 19. The results showed that administration by intraperitoneal injection in HB0017 + HB0034SA group could significantly inhibit renal fibrosis in NZB/NZW F1 mice.

### Example 6 Therapeutic effect of the combination of IL-17A antibody and IL-36R antibody on lupus nephritis in spontaneous lupus erythematosus mice (MRL/lpr)

30 MRL/lpr mice were randomly divided into 2 groups according to the animal ANA level and anti-dsDNA level, with 15 mice in each group, namely the HB0017 + HB0034SA combination group (25 + 25 mg/kg, i.p.) and the isotype control group (IgG1 50 mg/kg, i.p.). When MRL/lpr mice were 10 weeks old, the animals were administered by intraperitoneal injection. The frequency of administration was twice a week, and the administration lasted for 10 to 20 weeks, and then the animals were observed for another 2 weeks. The body weight was measured once a week, random urine was collected once a week to detect urine protein concentration, and ANA and anti-dsDNA IgG in plasma were tested monthly. At the end of the experiment, the left kidney of the animal was collected, fixed and sliced for Masson staining and fibrosis scoring.

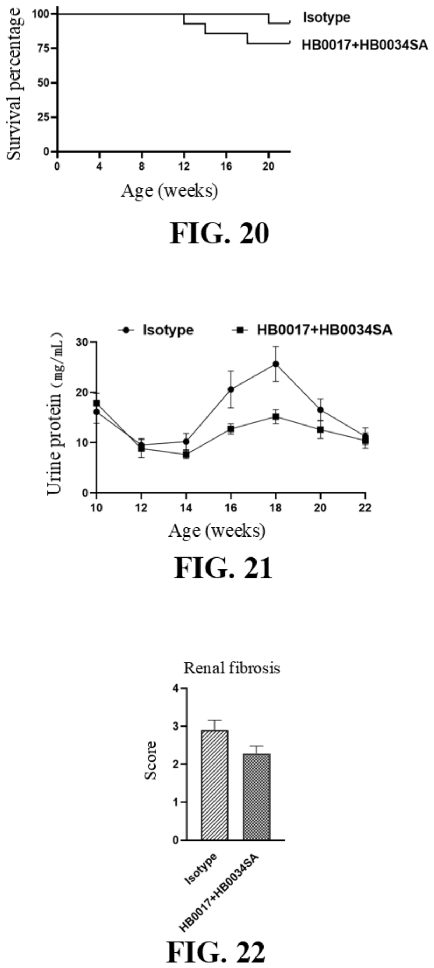

The survival rate statistics of MRL/lpr mice are shown in FIG. 20. The results showed that administration by intraperitoneal injection in HB0017 + HB0034SA group reduced the mortality rate of MRL/lpr mice.

The results of urine protein level detection of MRL/lpr mice are shown in FIG. 21. The results showed that administration by intraperitoneal injection in HB0017 + HB0034SA group inhibited the urine protein level in MRL/lpr mice.

The results of renal fibrosis evaluation of MRL/lpr mice are shown in FIG. 22. The results showed that administration by intraperitoneal injection in HB0017 + HB0034SA group could inhibit renal fibrosis in MRL/lpr mice.

## Claims

1. Use of a drug combination in the preparation of a drug for preventing and/or treating systemic lupus erythematosus, wherein the drug combination comprises an antigen binding protein targeting IL-17A and an antigen binding protein targeting IL-36R.

2. The use according to claim 1, wherein the IL-17A is human IL-17A.

3. The use according to any one of claims 1-2, wherein the antigen binding protein targeting IL-17A is capable of blocking the related effects of IL-17A and IL-17R.

4. The use according to any one of claims 1-3, wherein the antigen binding protein targeting IL-17A comprises at least one CDR from an antibody heavy chain variable region VH, and the VH comprises an amino acid sequence as set forth in SEQ ID NO: 16.

5. The use according to any one of claims 1-4, wherein the antigen binding protein targeting IL-17A comprises HCDR3, and the HCDR3 comprises an amino acid sequence as set forth in SEQ ID NO: 13.

6. The use according to any one of claims 1-5, wherein the antigen binding protein targeting IL-17A comprises HCDR2, and the HCDR2 comprises an amino acid sequence as set forth in SEQ ID NO: 14.

7. The use according to any one of claims 1-6, wherein the antigen binding protein targeting IL-17A comprises HCDR1, and the HCDR1 comprises an amino acid sequence as set forth in SEQ ID NO: 15.

8. The use according to any one of claims 1-7, wherein the antigen binding protein targeting IL-17A comprises VH, and the VH comprises an amino acid sequence as set forth in SEQ ID NO: 16.

9. The use according to any one of claims 1-8, wherein the antigen binding protein targeting IL-17A comprises an antibody heavy chain constant region.

10. The use according to claim 9, wherein the antibody heavy chain constant region is derived from a heavy chain constant region of IgG.

11. The use according to any one of claims 9-10, wherein the antibody heavy chain constant region is derived from a heavy chain constant region of IgG1 or IgG4.

12. The use according to any one of claims 9-11, wherein the heavy chain constant region of the antigen binding protein targeting IL-17A comprises an amino acid sequence as set forth in SEQ ID NO: 21.

13. The use according to any one of claims 1-12, wherein the antigen binding protein targeting IL-17A comprises at least one CDR from an antibody light chain variable region, and the VL comprises an amino acid sequence as set forth in SEQ ID NO: 20.

14. The use according to any one of claims 1-13, wherein the antigen binding protein targeting IL-17A comprises LCDR3, and the LCDR3 comprises an amino acid sequence as set forth in SEQ ID NO: 17.

15. The use according to any one of claims 1-14, wherein the antigen binding protein targeting IL-17A comprises LCDR2, and the LCDR2 comprises an amino acid sequence as set forth in SEQ ID NO: 18 (KVS).

16. The use according to any one of claims 1-15, wherein the antigen binding protein targeting IL-17A comprises LCDR1, and the LCDR1 comprises an amino acid sequence as set forth in SEQ ID NO: 19.

17. The use according to any one of claims 1-16, wherein the antigen binding protein targeting IL-17A comprises VL, and the VL comprises an amino acid sequence as set forth in SEQ ID NO: 20.

18. The use according to any one of claims 1-17, wherein the antigen binding protein targeting IL-17A comprises a light chain constant region.

19. The use according to claim 18, wherein the light chain constant region is derived from Igκ.

20. The use according to any one of claims 18-19, wherein the light chain constant region of the antigen binding protein targeting IL-17A comprises an amino acid sequence as set forth in SEQ ID NO: 10.

21. The use according to any one of claims 1-20, wherein the IL-36R is human IL-36R.

22. The use according to any one of claims 1-21, wherein the antigen binding protein targeting IL-36R comprises at least one CDR from an antibody heavy chain variable region VH, and the VH comprises an amino acid sequence as set forth in SEQ ID NO: 4 or SEQ ID NO: 30.

23. The use according to any one of claims 1-22, wherein the antigen binding protein targeting IL-36R comprises HCDR3, and the HCDR3 comprises an amino acid sequence as set forth in SEQ ID NO: 1 or SEQ ID NO: 27.

24. The use according to any one of claims 1-23, wherein the antigen binding protein targeting IL-36R comprises HCDR2, and the HCDR2 comprises an amino acid sequence as set forth in SEQ ID NO: 2 or SEQ ID NO: 28.

25. The use according to any one of claims 1-24, wherein the antigen binding protein targeting IL-36R comprises HCDR1, and the HCDR1 comprises an amino acid sequence as set forth in SEQ ID NO: 3 or SEQ ID NO: 29.

26. The use according to any one of claims 1-25, wherein the antigen binding protein targeting IL-36R comprises VH, and the VH comprises an amino acid sequence as set forth in SEQ ID NO: 4 or SEQ ID NO: 30.

27. The use according to any one of claims 1-26, wherein the antigen binding protein targeting IL-36R comprises an antibody heavy chain constant region.

28. The use according to claim 27, wherein the antibody heavy chain constant region is derived from a heavy chain constant region of IgG.

29. The use according to any one of claims 27-28, wherein the antibody heavy chain constant region is derived from a heavy chain constant region of IgG1.

30. The use according to any one of claims 27-29, wherein the heavy chain constant region of the antigen binding protein targeting IL-36R comprises an amino acid sequence as set forth in SEQ ID NO: 9 or SEQ ID NO: 35.

31. The use according to any one of claims 1-30, wherein the antigen binding protein targeting IL-36R comprises at least one CDR from an antibody light chain variable region, and the VL comprises an amino acid sequence as set forth in SEQ ID NO: 8 or SEQ ID NO: 34.

32. The use according to any one of claims 1-31, wherein the antigen binding protein targeting IL-36R comprises LCDR3, and the LCDR3 comprises an amino acid sequence as set forth in SEQ ID NO: 5 or SEQ ID NO: 31.

33. The use according to any one of claims 1-32, wherein the antigen binding protein targeting IL-36R comprises LCDR2, and the LCDR2 comprises an amino acid sequence as set forth in SEQ ID NO: 6 or SEQ ID NO: 32 (AAS).

34. The use according to any one of claims 1-33, wherein the antigen binding protein targeting IL-36R comprises LCDR1, and the LCDR1 comprises an amino acid sequence as set forth in SEQ ID NO: 7 or SEQ ID NO: 33.

35. The use according to any one of claims 1-34, wherein the antigen binding protein targeting IL-36R comprises VL, and the VL comprises an amino acid sequence as set forth in SEQ ID NO: 8 or SEQ ID NO: 34.

36. The use according to any one of claims 1-35, wherein the antigen binding protein targeting IL-36R comprises a light chain constant region.

37. The use according to claim 36, wherein the light chain constant region is derived from Igκ.

38. The use according to any one of claims 36-37, wherein the light chain constant region of the antigen binding protein targeting IL-36R comprises an amino acid sequence as set forth in SEQ ID NO: 10 or SEQ ID NO: 36.

39. The use according to any one of claims 1-38, wherein the systemic lupus erythematosus includes systemic lupus erythematosus nephritis.

40. Use of an isolated antigen binding protein in the preparation of a drug, wherein the isolated antigen binding protein comprises a first binding domain targeting IL-17A, and a second binding domain targeting IL-36R, and the drug is used to prevent and/or treat systemic lupus erythematosus.

41. The use according to claim 40, wherein the isolated antigen binding protein has one or more of the following properties:
(1) binding to IL-36R with high affinity;
(2) binding to IL-1Rrp2 with high affinity;
(3) blocking the binding of IL-36α, IL-36β, and IL-36γ to IL-36R; and
(4) blocking IL-36α-, IL-36β-, and IL-36γ-mediated cytokine secretion.

42. The use according to any one of claims 40-41, wherein the isolated antigen binding protein is selected from a monoclonal antibody, a chimeric antibody, a humanized antibody, or a combination thereof.

43. The use according to any one of claims 40-42, wherein the first binding domain of the isolated antigen binding protein comprises at least one CDR from an antibody heavy chain variable region VH, and the VH comprises an amino acid sequence as set forth in SEQ ID NO: 16.

44. The use according to any one of claims 40-43, wherein the first binding domain of the isolated antigen binding protein comprises HCDR3, and the HCDR3 comprises an amino acid sequence as set forth in SEQ ID NO: 13.

45. The use according to any one of claims 40-44, wherein the first binding domain of the isolated antigen binding protein comprises HCDR2, and the HCDR2 comprises an amino acid sequence as set forth in SEQ ID NO: 14.

46. The use according to any one of claims 40-45, wherein the first binding domain of the isolated antigen binding protein comprises HCDR1, and the HCDR1 comprises an amino acid sequence as set forth in SEQ ID NO: 15.

47. The use according to any one of claims 40-46, wherein the first binding domain of the isolated antigen binding protein comprises VH, and the VH comprises an amino acid sequence as set forth in SEQ ID NO: 16.

48. The use according to any one of claims 40-47, wherein the first binding domain of the isolated antigen binding protein comprises at least one CDR from an light chain variable region VL, and the VL comprises an amino acid sequence as set forth in SEQ ID NO: 20.

49. The use according to any one of claims 40-48, wherein the first binding domain of the isolated antigen binding protein comprises LCDR3, and the LCDR3 comprises an amino acid sequence as set forth in SEQ ID NO: 17.

50. The use according to any one of claims 40-49, wherein the first binding domain of the isolated antigen binding protein comprises LCDR2, and the LCDR2 comprises an amino acid sequence as set forth in SEQ ID NO: 18 (KVS).

51. The use according to any one of claims 40-50, wherein the first binding domain of the isolated antigen binding protein comprises LCDR1, and the LCDR1 comprises an amino acid sequence as set forth in SEQ ID NO: 19.

52. The use according to any one of claims 40-51, wherein the first binding domain of the isolated antigen binding protein comprises VL, and the VL comprises an amino acid sequence as set forth in SEQ ID NO: 20.

53. The use according to any one of claims 40-52, wherein the isolated antigen binding protein comprises a heavy chain constant region.

54. The use according to any one of claims 40-53, wherein the isolated antigen binding protein comprises a light chain constant region.

55. The use according to any one of claims 53-54, wherein the heavy chain constant region is derived from human IgG1 or human IgG4.

56. The use according to any one of claims 54-55, wherein the light chain constant region is derived from human IgG1 or human IgG4.

57. The use according to any one of claims 53-56, wherein the heavy chain constant region comprises an amino acid sequence as set forth in SEQ ID NO: 21.

58. The use according to any one of claims 54-57, wherein the light chain constant region comprises an amino acid sequence as set forth in SEQ ID NO: 10.

59. The use according to any one of claims 40-58, wherein the second binding domain of the isolated antigen binding protein comprises at least one CDR from an antibody heavy chain variable region VH, and the VH comprises an amino acid sequence as set forth in SEQ ID NO: 4 or SEQ ID NO: 30.

60. The use according to any one of claims 40-59, wherein the second binding domain of the isolated antigen binding protein comprises HCDR3, and the HCDR3 comprises an amino acid sequence as set forth in SEQ ID NO: 1 or SEQ ID NO: 27.

61. The use according to any one of claims 40-60, wherein the second binding domain of the isolated antigen binding protein comprises HCDR2, and the HCDR2 comprises an amino acid sequence as set forth in SEQ ID NO: 2 or SEQ ID NO: 28.

62. The use according to any one of claims 40-61, wherein the second binding domain of the isolated antigen binding protein comprises HCDR1, and the HCDR1 comprises an amino acid sequence as set forth in SEQ ID NO: 3 or SEQ ID NO: 29.

63. The use according to any one of claims 40-62, wherein the second binding domain of the isolated antigen binding protein comprises VH, and the VH comprises an amino acid sequence as set forth in SEQ ID NO: 4 or SEQ ID NO: 30.

64. The use according to any one of claims 40-63, wherein the second binding domain of the isolated antigen binding protein comprises at least one CDR from an antibody light chain variable region VL, and the VL comprises an amino acid sequence as set forth in SEQ ID NO: 8 or SEQ ID NO: 34.

65. The use according to any one of claims 40-64, wherein the second binding domain of the isolated antigen binding protein comprises LCDR3, and the LCDR3 comprises an amino acid sequence as set forth in SEQ ID NO: 5 or SEQ ID NO: 31.

66. The use according to any one of claims 40-65, wherein the second binding domain of the isolated antigen binding protein comprises LCDR2, and the LCDR2 comprises an amino acid sequence as set forth in SEQ ID NO: 6 or SEQ ID NO: 32 (AAS).

67. The use according to any one of claims 40-66, wherein the second binding domain of the isolated antigen binding protein comprises LCDR1, and the LCDR1 comprises an amino acid sequence as set forth in SEQ ID NO: 7 or SEQ ID NO: 33.

68. The use according to any one of claims 40-67, wherein the second binding domain of the isolated antigen binding protein comprises VL, and the VL comprises an amino acid sequence as set forth in SEQ ID NO: 8 or SEQ ID NO: 34.

69. The use according to any one of claims 64-68, wherein the second binding domain of the isolated antigen binding protein comprises VH and VL, and the VH and VL are linked via a linking peptide.

70. The use according to claim 69, wherein the amino acid sequence of the linking peptide is (G4S)n, wherein n is a positive integer.

71. The use according to any one of claims 40-70, wherein the second binding domain of the isolated antigen binding protein is scFv.

72. The use according to claim 71, wherein the scFv of the second binding domain of the isolated antigen binding protein comprises an amino acid sequence as set forth in SEQ ID NO: 24.

73. The use according to any one of claims 40-72, wherein the isolated antigen binding protein comprises: (a) a first binding domain of an anti-IL-17A antibody; and (b) a second binding domain linked to the anti-IL-17A antibody.

74. The use according to claim 73, wherein the second binding domain is a single-chain variable region (scFv) of an anti-IL-36R antibody.

75. The use according to any one of claims 40-74, wherein the second binding domain is directly or indirectly linked to the first binding domain.

76. The use according to claim 75, wherein the second binding domain is linked to the first binding domain via a linker.

77. The use according to any one of claims 73-76, wherein the anti-IL-17A antibody and the scFv of the anti-IL-36R antibody are linked via a linker sequence.

78. The use according to any one of claims 40-77, wherein the isolated antigen binding protein is a homodimer.

79. The use according to any one of claims 40-78, wherein the isolated antigen binding protein is a tetravalent antibody.

80. The use according to any one of claims 40-79, wherein the isolated antigen binding protein is a bispecific antibody.

81. The use according to claim 80, wherein the bispecific antibody is a dual-chain antibody or a single-chain antibody.

82. The use according to any one of claims 80-81, wherein the bispecific antibody has a structure represented by formula I from N-terminus to C-terminus: 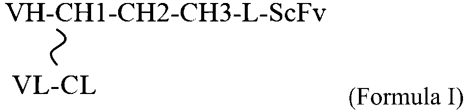 wherein,
VH represents the heavy chain variable region of the anti-IL-17A antibody;
VL represents the light chain variable region of the anti-IL-17A antibody;
CH1, CH2 and CH3 represent the heavy chain constant regions CH1, CH2 and CH3 of the anti-IL-17A antibody, respectively;
CL represents the light chain constant region of the anti-IL-17A antibody;
ScFv represents the ScFv of the anti-IL-36R antibody;
L is a linker element;
"~" represents a disulfide bond;
"-" represents a peptide bond;
wherein, the bispecific antibody has an activity of binding to IL-17A and IL-36R simultaneously.

83. The use according to any one of claims 80-82, wherein the bispecific antibody is formed by fusion of the anti-IL-17A antibody and the ScFv of the anti-IL-36R antibody, and has two pairs of peptide chains that are symmetrical to each other; each pair of peptide chains comprises a light chain L chain and a heavy chain H chain; all the peptide chains are linked via disulfide bonds, and any pair of peptide chains has a structure of the H chain and the L chain represented by formula I from N-terminus to C-terminus.

84. The use according to any one of claims 80-83, wherein the bispecific antibody is a homodimer of the peptide chain of the structure represented by formula I.

85. The use according to any one of claims 82-84, wherein the linker element is a linker peptide.

86. The use according to claim 85, wherein the amino acid sequence of the linking peptide is (G4S)n, wherein n is a positive integer.

87. The use according to any one of claims 83-86, wherein the H chain of the bispecific antibody has an amino acid sequence as set forth in SEQ ID NO: 25; and the L chain of the bispecific antibody has an amino acid sequence as set forth in SEQ ID NO: 23.

88. The use according to any one of claims 1-87, wherein the isolated antigen binding protein further comprises a detectable marker, a targeting marker, a drug, a toxin, a cytokine, a radionuclide, or an enzyme.

89. The use according to any one of claims 1-88, wherein the systemic lupus erythematosus comprises lupus erythematosus nephritis.

90. The use according to any one of claims 1-89, wherein the drug is capable of alleviating the elevated urine protein level caused by systemic lupus erythematosus.

91. The use according to any one of claims 1-90, wherein the drug is capable of alleviating renal pathological damage caused by systemic lupus erythematosus.

92. The use according to any one of claims 1-91, wherein the drug is capable of alleviating and/or inhibiting renal fibrosis caused by systemic lupus erythematosus.

93. A method for preventing and/or treating systemic lupus erythematosus, comprising administering a drug to a subject in need thereof, wherein the drug is capable of targeting IL-17A and IL-36R.

94. The method according to claim 93, wherein the systemic lupus erythematosus comprises lupus erythematosus nephritis.

95. A method for alleviating elevated urine protein level caused by systemic lupus erythematosus, comprising administering a drug to a subject in need thereof, wherein the drug is capable of targeting IL-17A and IL-36R.

96. A method for alleviating renal pathological damage caused by systemic lupus erythematosus, comprising administering a drug to a subject in need thereof, wherein the drug is capable of targeting IL-17A and IL-36R.

97. A method for alleviating and/or inhibiting renal fibrosis caused by systemic lupus erythematosus, comprising administering a drug to a subject in need thereof, wherein the drug is capable of targeting IL-17A and IL-36R.

98. The method according to any one of claims 93-97, wherein the IL-17A is human IL-17A.

99. The method according to any one of claims 93-98, wherein the IL-36R is human IL-36R.

100. The method according to any one of claims 93-99, wherein the drug comprises an antigen binding protein targeting IL-17A, and an antigen binding protein targeting IL-36R.

101. The method according to claim 100, wherein the antigen binding protein targeting IL-17A is capable of blocking the related effects of IL-17A and IL-17R.

102. The method according to any one of claims 100-101, wherein the antigen binding protein targeting IL-17A comprises at least one CDR from an antibody heavy chain variable region VH, and the VH comprises an amino acid sequence as set forth in SEQ ID NO: 16.

103. The method according to any one of claims 100-102, wherein the antigen binding protein targeting IL-17A comprises HCDR3, and the HCDR3 comprises an amino acid sequence as set forth in SEQ ID NO: 13.

104. The method according to any one of claims 100-103, wherein the antigen binding protein targeting IL-17A comprises HCDR2, and the HCDR2 comprises an amino acid sequence as set forth in SEQ ID NO: 14.

105. The method according to any one of claims 100-104, wherein the antigen binding protein targeting IL-17A comprises HCDR1, and the HCDR1 comprises an amino acid sequence as set forth in SEQ ID NO: 15.

106. The method according to any one of claims 100-105, wherein the antigen binding protein targeting IL-17A comprises VH, and the VH comprises an amino acid sequence as set forth in SEQ ID NO: 16.

107. The method according to any one of claims 100-106, wherein the antigen binding protein targeting IL-17A comprises an antibody heavy chain constant region.

108. The method according to claim 107, wherein the antibody heavy chain constant region is derived from a heavy chain constant region of IgG.

109. The method according to any one of claims 107-108, wherein the antibody heavy chain constant region is derived from a heavy chain constant region of IgG1.

110. The method according to any one of claims 107-109, wherein the antibody heavy chain constant region comprises an amino acid sequence as set forth in SEQ ID NO: 21.

111. The method according to any one of claims 100-110, wherein the antigen binding protein targeting IL-17A comprises at least one CDR from an antibody light chain variable region, and the VL comprises an amino acid sequence as set forth in SEQ ID NO: 20.

112. The method according to any one of claims 100-111, wherein the antigen binding protein targeting IL-17A comprises LCDR3, and the LCDR3 comprises an amino acid sequence as set forth in SEQ ID NO: 17.

113. The method according to any one of claims 100-112, wherein the antigen binding protein targeting IL-17A comprises LCDR2, and the LCDR2 comprises an amino acid sequence as set forth in SEQ ID NO: 18 (KVS).

114. The method according to any one of claims 100-113, wherein the antigen binding protein targeting IL-17A comprises LCDR1, and the LCDR1 comprises an amino acid sequence as set forth in SEQ ID NO: 19.

115. The method according to any one of claims 100-114, wherein the antigen binding protein targeting IL-17A comprises VL, and the VL comprises an amino acid sequence as set forth in SEQ ID NO: 20.

116. The method according to any one of claims 100-115, wherein the antigen binding protein targeting IL-17A comprises a light chain constant region.

117. The method according to claim 116, wherein the light chain constant region is derived from Igκ.

118. The method according to any one of claims 116-117, wherein the light chain constant region comprises an amino acid sequence as set forth in SEQ ID NO: 10.

119. The method according to any one of claims 100-118, wherein the antigen binding protein targeting IL-36R comprises at least one CDR from an antibody heavy chain variable region VH, and the VH comprises an amino acid sequence as set forth in SEQ ID NO: 4 or SEQ ID NO: 30.

120. The method according to any one of claims 100-119, wherein the antigen binding protein targeting IL-36R comprises HCDR3, and the HCDR3 comprises an amino acid sequence as set forth in SEQ ID NO: 1 or SEQ ID NO: 27.

121. The method according to any one of claims 100-120, wherein the antigen binding protein targeting IL-36R comprises HCDR2, and the HCDR2 comprises an amino acid sequence as set forth in SEQ ID NO: 2 or SEQ ID NO: 28.

122. The method according to any one of claims 100-121, wherein the antigen binding protein targeting IL-36R comprises HCDR1, and the HCDR1 comprises an amino acid sequence as set forth in SEQ ID NO: 3 or SEQ ID NO: 29.

123. The method according to any one of claims 100-122, wherein the antigen binding protein targeting IL-36R comprises VH, and the VH comprises an amino acid sequence as set forth in SEQ ID NO: 4 or SEQ ID NO: 30.

124. The method according to any one of claims 100-123, wherein the antigen binding protein targeting IL-36R comprises an antibody heavy chain constant region.

125. The method according to claim 124, wherein the antibody heavy chain constant region is derived from a heavy chain constant region of IgG.

126. The method according to any one of claims 124-125, wherein the antibody heavy chain constant region is derived from a heavy chain constant region of IgG1.

127. The method according to any one of claims 124-126, wherein the antibody heavy chain constant region comprises an amino acid sequence as set forth in SEQ ID NO: 9 or SEQ ID NO: 35.

128. The method according to any one of claims 100-127, wherein the antigen binding protein targeting IL-36R comprises at least one CDR from an antibody light chain variable region, and the VL comprises an amino acid sequence as set forth in SEQ ID NO: 8 or SEQ ID NO: 34.

129. The method according to any one of claims 100-128, wherein the antigen binding protein targeting IL-36R comprises LCDR3, and the LCDR3 comprises an amino acid sequence as set forth in SEQ ID NO: 5 or SEQ ID NO: 31.

130. The method according to any one of claims 100-129, wherein the antigen binding protein targeting IL-36R comprises LCDR2, and the LCDR2 comprises an amino acid sequence as set forth in SEQ ID NO: 6 (STS) or SEQ ID NO: 32 (AAS).

131. The method according to any one of claims 100-130, wherein the antigen binding protein targeting IL-36R comprises LCDR1, and the LCDR1 comprises an amino acid sequence as set forth in SEQ ID NO: 7 or SEQ ID NO: 33.

132. The method according to any one of claims 100-131, wherein the antigen binding protein targeting IL-36R comprises VL, and the VL comprises an amino acid sequence as set forth in SEQ ID NO: 8 or SEQ ID NO: 34.

133. The method according to any one of claims 100-132, wherein the antigen binding protein targeting IL-36R comprises a light chain constant region.

134. The method according to claim 133, wherein the light chain constant region is derived from Igκ.

135. The method according to any one of claims 133-134, wherein the light chain constant region comprises an amino acid sequence as set forth in SEQ ID NO: 10 or SEQ ID NO: 36.

136. The method according to any one of claims 93-135, wherein the drug comprises an isolated antigen binding protein, and the isolated antigen binding protein comprises a first binding domain targeting IL-17A, and a second binding domain targeting IL-36R.

137. The method according to claim 136, wherein the isolated antigen binding protein has one or more of the following properties:
(1) binding to IL-36R with high affinity;
(2) binding to IL-1Rrp2 with high affinity;
(3) blocking the binding of IL-36α, IL-36β, and IL-36γ to IL-36R; and
(4) blocking IL-36α-, IL-36β-, and IL-36γ-mediated cytokine secretion.

138. The method according to any one of claims 136-137, wherein the isolated antigen binding protein is selected from a monoclonal antibody, a chimeric antibody, a humanized antibody, or a combination thereof.

139. The method according to any one of claims 136-138, wherein the first binding domain of the isolated antigen binding protein comprises at least one CDR from an antibody heavy chain variable region VH, and the VH comprises an amino acid sequence as set forth in SEQ ID NO: 16.

140. The method according to any one of claims 136-139, wherein the first binding domain of the isolated antigen binding protein comprises HCDR3, and the HCDR3 comprises an amino acid sequence as set forth in SEQ ID NO: 13.

141. The method according to any one of claims 136-140, wherein the first binding domain of the isolated antigen binding protein comprises HCDR2, and the HCDR2 comprises an amino acid sequence as set forth in SEQ ID NO: 14.

142. The method according to any one of claims 136-141, wherein the first binding domain of the isolated antigen binding protein comprises HCDR1, and the HCDR1 comprises an amino acid sequence as set forth in SEQ ID NO: 15.

143. The method according to any one of claims 136-142, wherein the first binding domain of the isolated antigen binding protein comprises VH, and the VH comprises an amino acid sequence as set forth in SEQ ID NO: 16.

144. The method according to any one of claims 136-143, wherein the first binding domain of the isolated antigen binding protein comprises at least one CDR from an light chain variable region VL, and the VL comprises an amino acid sequence as set forth in SEQ ID NO: 20.

145. The method according to any one of claims 136-144, wherein the first binding domain of the isolated antigen binding protein comprises LCDR3, and the LCDR3 comprises an amino acid sequence as set forth in SEQ ID NO: 17.

146. The method according to any one of claims 136-145, wherein the first binding domain of the isolated antigen binding protein comprises LCDR2, and the LCDR2 comprises an amino acid sequence as set forth in SEQ ID NO: 18 (KVS).

147. The method according to any one of claims 136-146, wherein the first binding domain of the isolated antigen binding protein comprises LCDR1, and the LCDR1 comprises an amino acid sequence as set forth in SEQ ID NO: 19.

148. The method according to any one of claims 136-147, wherein the first binding domain of the isolated antigen binding protein comprises VL, and the VL comprises an amino acid sequence as set forth in SEQ ID NO: 20.

149. The method according to any one of claims 136-148, wherein the isolated antigen binding protein comprises a heavy chain constant region.

150. The method according to any one of claims 136-149, wherein the isolated antigen binding protein comprises a light chain constant region.

151. The method according to any one of claims 149-150, wherein the heavy chain constant region is derived from human IgG1 or human IgG4.

152. The method according to any one of claims 150-151, wherein the light chain constant region is derived from human IgG1 or human IgG4.

153. The method according to any one of claims 149-152, wherein the heavy chain constant region comprises an amino acid sequence as set forth in SEQ ID NO: 21.

154. The method according to any one of claims 150-153, wherein the light chain constant region comprises an amino acid sequence as set forth in SEQ ID NO: 10.

155. The method according to any one of claims 136-154, wherein the second binding domain of the isolated antigen binding protein comprises at least one CDR from an antibody heavy chain variable region VH, and the VH comprises an amino acid sequence as set forth in SEQ ID NO: 4 or SEQ ID NO: 30.

156. The method according to any one of claims 136-155, wherein the second binding domain of the isolated antigen binding protein comprises HCDR3, and the HCDR3 comprises an amino acid sequence as set forth in SEQ ID NO: 1 or SEQ ID NO: 27.

157. The method according to any one of claims 136-156, wherein the second binding domain of the isolated antigen binding protein comprises HCDR2, and the HCDR2 comprises an amino acid sequence as set forth in SEQ ID NO: 2 or SEQ ID NO: 28.

158. The method according to any one of claims 136-157, wherein the second binding domain of the isolated antigen binding protein comprises HCDR1, and the HCDR1 comprises an amino acid sequence as set forth in SEQ ID NO: 3 or SEQ ID NO: 29.

159. The method according to any one of claims 136-158, wherein the second binding domain of the isolated antigen binding protein comprises VH, and the VH comprises an amino acid sequence as set forth in SEQ ID NO: 4 or SEQ ID NO: 30.

160. The method according to any one of claims 136-159, wherein the second binding domain of the isolated antigen binding protein comprises at least one CDR from an antibody light chain variable region VL, and the VL comprises an amino acid sequence as set forth in SEQ ID NO: 8 or SEQ ID NO: 34.

161. The method according to any one of claims 136-160, wherein the second binding domain of the isolated antigen binding protein comprises LCDR3, and the LCDR3 comprises an amino acid sequence as set forth in SEQ ID NO: 5 or SEQ ID NO: 31.

162. The method according to any one of claims 136-161, wherein the second binding domain of the isolated antigen binding protein comprises LCDR2, and the LCDR2 comprises an amino acid sequence as set forth in SEQ ID NO: 6 (STS) or SEQ ID NO: 32 (AAS).

163. The method according to any one of claims 136-162, wherein the second binding domain of the isolated antigen binding protein comprises LCDR1, and the LCDR1 comprises an amino acid sequence as set forth in SEQ ID NO: 7 or SEQ ID NO: 33.

164. The method according to any one of claims 136-163, wherein the second binding domain of the isolated antigen binding protein comprises VL, and the VL comprises an amino acid sequence as set forth in SEQ ID NO: 8 or SEQ ID NO: 34.

165. The method according to any one of claims 136-164, wherein the second binding domain of the isolated antigen binding protein comprises VH and VL, and the VH and VL are linked via a linking peptide.

166. The method according to claim 165, wherein the amino acid sequence of the linking peptide is (G4S)n, wherein n is a positive integer.

167. The method according to any one of claims 136-166, wherein the second binding domain of the isolated antigen binding protein is scFv.

168. The method according to claim 167, wherein the scFv of the second binding domain of the isolated antigen binding protein comprises an amino acid sequence as set forth in SEQ ID NO: 24.

169. The method according to any one of claims 136-168, wherein the isolated antigen binding protein comprises: (a) a first binding domain of an anti-IL-17A antibody; and (b) a second binding domain linked to the anti-IL-17A antibody.

170. The method according to claim 169, wherein the second binding domain is a single-chain variable region (scFv) of an anti-IL-36R antibody.

171. The method according to any one of claims 136-170, wherein the second binding domain is directly or indirectly linked to the first binding domain.

172. The method according to claim 171, wherein the second binding domain is linked to the first binding domain via a linker.

173. The method according to any one of claims 170-172, wherein the anti-IL-17A antibody and the scFv of the anti-IL-36R antibody are linked via a linker sequence.

174. The method according to any one of claims 136-173, wherein the isolated antigen binding protein is a homodimer.

175. The method according to any one of claims 136-174, wherein the isolated antigen binding protein is a tetravalent antibody.

176. The method according to any one of claims 136-175, wherein the isolated antigen binding protein is a bispecific antibody.

177. The method according to claim 176, wherein the bispecific antibody is a dual-chain antibody or a single-chain antibody.

178. The method according to any one of claims 176-177, wherein the bispecific antibody has a structure represented by formula I from N-terminus to C-terminus: 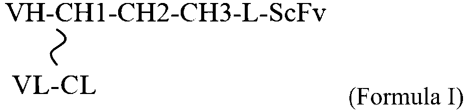 wherein,
VH represents the heavy chain variable region of the anti-IL-17A antibody;
VL represents the light chain variable region of the anti-IL-17A antibody;
CH1, CH2 and CH3 represent the heavy chain constant regions CH1, CH2 and CH3 of the anti-IL-17A antibody, respectively;
CL represents the light chain constant region of the anti-IL-17A antibody;
ScFv represents the ScFv of the anti-IL-36R antibody;
L is a linker element;
"~" represents a disulfide bond;
"-" represents a peptide bond;
wherein, the bispecific antibody has an activity of binding to IL-17A and IL-36R simultaneously.

179. The method according to any one of claims 176-178, wherein the bispecific antibody is formed by fusion of the anti-IL-17A antibody and the ScFv of the anti-IL-36R antibody, and has two pairs of peptide chains that are symmetrical to each other; each pair of peptide chains comprises a light chain L chain and a heavy chain H chain; all the peptide chains are linked via disulfide bonds, and any pair of peptide chains has a structure of the H chain and the L chain represented by formula I from N-terminus to C-terminus.

180. The method according to any one of claims 178-179, wherein the bispecific antibody is a homodimer of the peptide chain of the structure represented by formula I.

181. The method according to any one of claims 178-180, wherein the linker element is a linker peptide.

182. The method according to claim 181, wherein the amino acid sequence of the linking peptide is (G4S)n, wherein n is a positive integer.

183. The method according to any one of claims 179-182, wherein the H chain of the bispecific antibody has an amino acid sequence as set forth in SEQ ID NO: 25; and the L chain of the bispecific antibody has an amino acid sequence as set forth in SEQ ID NO: 23.

184. The method according to any one of claims 100-183, wherein the isolated antigen binding protein further comprises a detectable marker, a targeting marker, a drug, a toxin, a cytokine, a radionuclide, or an enzyme.

185. The method according to any one of claims 93-184, wherein the drug further optionally comprises a pharmaceutically acceptable carrier.

186. The method according to any one of claims 93-185, wherein the drug comprises a nucleic acid molecule encoding the antigen binding protein.
